# EUROPEAN PATENT APPLICATION

(11) **EP 3 192 517 A1**
(43) Date of publication of application: **19.07.2017**
(21) Application number: 15833898.8
(22) Date of filing: 17.08.2015
(51) Int. Cl.: A61K 35/17, A61P 31/00, A61P 35/00, A61P 37/02, A61P 37/04, G01N 33/48, G01N 33/53

(54) **METHOD FOR ENHANCING IMMUNE CELL FUNCTION AND METHOD FOR ASSESSING IMMUNE CELL MULTIFUNCTIONALITY**

(30) Priority: 19.08.2014 JP 2014166593; 20.04.2015 JP 2015085556
(60) Divisional application: 17174222.4
(71) Applicant: National University Corporation Okayama University, Okayama-shi, Okayama 700-8530 (JP)
(72) Inventor: UDONO, Heiichiro, Okayama-shi Okayama 700-8530 (JP); EIKAWA, Shingo, Okayama-shi Okayama 700-8530 (JP); TOYOOKA, Shin-ichi, Okayama-shi Okayama 700-8530 (JP)
(74) Representative: Hoffmann Eitle
(86) International application number: PCT/JP2015/073011
(87) International publication number: WO 2016/027764

(57) **Abstract**

The present invention provides a method for enhancing immune cell function by activating various immune cells ex vivo and provides immune cells with enhanced function. The invention further provides an immune-related cell multifunctionality evaluation method. A biguanide antidiabetic drug selected from metformin, phenformin, and buformin is capable of enhancing immune cell multifunctionality by increasing CD8+T cells having a high ability to produce IL-2, TNFα, and IFNγ. The immune-related cell multifunctionality may be evaluated by comparing immune cells treated with a biguanide antidiabetic drug selected from metformin, phenformin, and buformin, with control immune cells untreated with the biguanide antidiabetic drug. When the multifunctionality of immune cells treated with the biguanide antidiabetic drug selected from metformin, phenformin, and buformin is determined to be significantly increased compared with the control, it can be evaluated that the sensitivity of the immune cells to the therapeutic agent is improved.

## Description

### Technical Field

The present invention relates to a method for enhancing functions of immune cells, immune cells with enhanced function, and an agent for treating and/or preventing diseases associated with immune abnormalities containing the immune cells with enhanced function as an active ingredient. The present invention further relates to a method for evaluating multifunctionality of immune cells.

### Background Art

T cells serve as immune cells and account for 70 to 80% of peripheral blood lymphocytes. T cells are distributed in the spleen or lymph nodes throughout the body and contribute to biological defense. T cells are classified into cytotoxic T cells (CD8⁺T cells), which directly affect virally infected cells or cancer cells, and into helper T cells (CD4⁺T cells), which assist the function of immunocompetent cells such as cytotoxic T cells. T cells have T cell receptors (TCR) expressed on their surfaces and recognize antigens by these TCR. A single T cell expresses TCR for recognizing one specific kind of antigen. This is called antigen specificity of T cells. T cells play an important role in various immune-related clinical conditions, including infections, tumors, organ transplants, and allergies.

In recent years, the mechanism of immune exhaustion has been clarified, and there are reports that inhibition of the bonds of exhaustion molecules and ligands is important in cancer treatments. Some cancer patients have significantly inferior immune response (immune exhaustion). Immune exhaustion is caused by the expression of exhaustion markers, such as PD-1, Tim-3, Lag3, and the like, by CD8⁺T cells, and these exhaustion marker molecules (exhaustion molecules) binding with its ligands, causing loss of the ability to simultaneously produce IL-2, TNFα, and IFNγ (multifunctionality), and further cause cell death by apoptosis (Non-Patent Documents 1 to 6). To avoid such apoptosis and immune exhaustion of CD8⁺T cells, it is necessary to suppress the expression itself of the exhaustion molecules. However, since there is no such technology at present, the inhibition of the bonds of the exhaustion molecules with the ligands are the only method to recover the functions of CD8⁺T cells and prevent the apoptosis. However, the kinds (the number) of the exhaustion molecules are liable to increase, and research seeking the appropriate number of exhaustion molecules and the ligands to be inhibited from bonding is still in progress. Further, since the method clearly has side effects, a method with fewer side effects has been desired.

For the monitoring of immune status in a diseased state or the evaluation of vaccine efficacy, quantitative and qualitative measurements of antigen specific T cells as the evaluation of immune cell multifunctionality are important. For patients under immunotherapy such as cancer vaccine therapy, it has been necessary to conduct antigen stimulation of removed cells in vitro for 1 or 2 weeks so as to judge the therapeutic effect. Examples of T cell function measurement methods include cytotoxic tests, ELISA methods for detecting cytokine production, methods for detecting molecules secreted from the entire cell group by fluorescent bead immunoassay, intracellular cytokine analysis (intracellular cytokine staining assay: ICS method) by using flow cytometer (FCM, FACS) technologies, and methods for detecting secretion of molecules at individual cell basis by the ELISPOT (enzyme-linked immunospot) method.

Although the previously known technologies for measuring T cell function enable precise analysis of individual T cell functions, the analysis results were not necessarily correlated with the prognosis. More specifically, it has been difficult to ascertain immune kinetics of healthy subjects or patients with, for example, refractory infections or cancers, and to predict the prognosis of treatment by an immune activator. In the previously known technologies for increasing T cell function, there has been a limit to significantly increasing acquired immunity of patients with, for example, refractory infections or cancers or healthy subjects and thereby cure the symptoms. In the first place, the conditions in immune kinetics evaluation for accurately verifying or predicting therapeutic effects were not good enough, and there were no methods available for comprehensively judging and evaluating the methods and technologies for increasing various T cell functions (methods for evaluating multifunctionality of immune cells involved in treatment prognosis).

For example, in administering vaccines, it is almost always necessary to administer an adjuvant together with the antigen, but the adjuvant may cause serious side effects. No adjuvant that is safe, effective, and applicable to all vaccines has been discovered so far. Further, in many cases of infection or cancer, sufficient protective immunity cannot be obtained by the application of vaccine despite the increase in CD8-positive killer T cells against the vaccine in the peripheral blood. Such a failure in vaccine application is presumably often due to immune exhaustion. Based on this standpoint, there have been no comprehensive immune kinetics evaluation technologies and T cell function increasing agents or methods as a combined technology of a cell multifunctionality increasing method by canceling immune exhaustion of various immune cells and a method of accurately and easily predicting and evaluating the resulting effects.

Patent Document 1 discloses that metformin serves to potentiate the effects of chemotherapy drugs, but the Examples of Patent Document 1 disclose that metformin itself does not have a tumor treating effect. Non-patent Document 7 discloses the action mechanism of metformin as a type II diabetes therapeutic agent.

### Citation List

### Patent Documents

Patent Document 1: JP2013-503171A

### Non-patent Documents

Non-patent Document 1: Proc Natl Acad Sci USA. 2002; 99: 12293-7
Non-patent Document 2: Cancer Res. 2004; 64: 1140-5
Non-patent Document 3: J Exp Med. 2010; 207: 2187-94
Non-patent Document 4: Trends Immunol. 2011; 32: 345-9
Non-patent Document 5: Cancer Res. 2011; 71: 3540-51
Non-patent Document 6: Nat Rev Cancer. 2012; 12: 252-64
Non-patent Document 7: Nature 2006; 439: 682-687

### Summary of Invention

### Technical Problem

An object of the present invention is to activate immune cells ex vivo and provide a method for enhancing immune cell function, as well as providing immune cells with enhanced function. Another object of the present invention is to provide a method for evaluating multifunctionality of immune-related cells.

### Solution to Problem

In order to attain the above objects, the present inventors focused attention on cytokines produced by CD8⁺T cells and immune cell functions, and conducted extensive research. As a result, the inventors found that a biguanide antidiabetic drug selected from metformin, phenformin, and buformin increases CD8⁺T cells with a high ability to produce IL-2, TNFα and IFNγ, thereby enhancing the function; with this finding, the inventors completed the present invention. Immune cell multifunctionality is evaluated by comparing the multifunctionality of immune cells treated with a biguanide antidiabetic drug selected from metformin, phenformin, and buformin, with the multifunctionality of control immune cells that were not treated with the biguanide antidiabetic drug. When the immune cells treated with a biguanide antidiabetic drug selected from metformin, phenformin, and buformin are confirmed to have significantly increased multifunctionality compared with that of the control, it can be determined that the sensitivity of immune cells with respect to the therapeutic agent was improved. In the immune cells that were determined to have increased sensitivity to the therapeutic agent by the evaluation method, a high therapeutic effect from a biguanide antidiabetic drug selected from metformin, phenformin, and buformin can be expected.

The present invention includes the following inventions.
1. A method for enhancing immune cell function, comprising treating removed immune cells in vitro using a therapeutic agent comprising, as an active ingredient, a biguanide antidiabetic drug selected from metformin, phenformin, and buformin.
2. The method for enhancing immune cell function according to Item 1, wherein the immune cells are selected from CD8⁺T cells, CD4⁺T cells, NK cells, γδT cells, NKT cells, B cells, and bone marrow cells.
3. Immune cells that are enhanced in function by the method of Item 1 or 2.
4. An agent for treating and/or preventing diseases associated with immune abnormalities, comprising, as an active ingredient, immune cells that are enhanced in function by the method of any one of Items 1 to 3.
5. The agent for treating and/or preventing diseases associated with immune abnormalities according to Item 4, wherein the diseases associated with immune abnormalities are selected from cancers, infections, and autoimmune diseases.
6. A method for evaluating multifunctionality of removed immune cells, comprising treating removed immune cells in vitro using a therapeutic agent comprising, as an active ingredient, a biguanide antidiabetic drug selected from metformin, phenformin, and buformin; measuring cytokine production ability for three kinds of cytokines, IL-2, TNFα, and IFNγ, of the treated immune cells; and measuring a cell-positivity ratio representing a ratio of cells in which cytokine production abilities with respect to the three kinds of cytokines are all positive.
7. The multifunctionality evaluation method according to Item 6, further comprising the step of measuring expressions of Tim-3 and/or PD-1 in the removed immune cells.
8. The multifunctionality evaluation method according to Item 6 or 7, wherein the immune cells are selected from CD8⁺T cells, CD4⁺T cells, NK cells, γδT cells, NKT cells, B cells, and bone marrow cells.
9. A test method for diseases associated with immune abnormalities, the method using the multifunctionality evaluation method of any one of Items 6 to 8.
10. The test method according to Item 9, wherein the test of diseases associated with immune abnormalities is a test supplementarily used to determine prediction of efficacy of a therapeutic agent for the disease, prediction of severity of the disease, prediction of prognosis of the disease, or prediction of onset of disease.
11. The test method according to Item 10, wherein the therapeutic agent for the disease is a therapeutic agent comprising, as an active ingredient, a biguanide antidiabetic drug selected from metformin, phenformin, and buformin.
12. An agent for treating and/or preventing diseases associated with immune abnormalities, comprising, as an active ingredient, a biguanide antidiabetic drug selected from metformin, phenformin, and buformin, the agent being used after a test using the test method according to any one of Items 9 to 11.
13. The agent for treating and/or preventing diseases associated with immune abnormalities according to Item 12, wherein the diseases associated with immune abnormalities are selected from cancers, infections, and autoimmune diseases.
14. An agent for treating and/or preventing diseases associated with immune abnormalities, comprising, as an active ingredient, a biguanide antidiabetic drug selected from metformin, phenformin, and buformin, wherein the agent is administered in combination with at least one therapeutic agent selected from immunosuppressive factor blocking agent and costimulatory receptor agonist.
15. An agent for treating and/or preventing diseases associated with immune abnormalities, comprising, as an active ingredient, at least one therapeutic agent selected from immunosuppressive factor blocking agent and costimulatory receptor agonist, wherein the agent is administered in combination with a biguanide antidiabetic drug selected from metformin, phenformin, and buformin.
16. The agent for treating and/or preventing diseases associated with immune abnormalities according to Item 14 or 15, wherein the immunosuppressive factor blocking agent is one or any combination of antibodies or fusion proteins selected from anti-CTLA-4 antibody, anti-PD-1 antibody, anti-PD-L1 antibody, anti-PD-L2 antibody, PD-L1 fusion protein, PD-L2 fusion protein, anti-Tim-3 antibody, anti-LAG-3 antibody, anti-BTLA antibody, and anti-VISTA antibody.
17. The agent for treating and/or preventing diseases associated with immune abnormalities according to Item 16, wherein the anti-CTLA-4 antibody is Ipilimumab or Tremelimumab, the anti-PD-1 antibody is Nivolumab, Pembrolizumab, PDR-001, REGN-2810, BGB-A317, or AMP-514, and the anti-PD-L1 antibody is Atezolizumab, Avelumab, Durvalumab, or BMS-936559, and the PD-L2 fusion protein is AMP-224.
18. The agent for treating and/or preventing diseases associated with immune abnormalities according to Item 14 or 15, wherein the costimulatory receptor agonist is an antibody or any combination of antibodies selected from anti-CD137 antibody, anti-OX40 antibody, anti-HVEM antibody, anti-CD27 antibody, anti-GITR antibody, and anti-CD28 antibody.
19. An agent for treating and/or preventing diseases associated with immune abnormalities, comprising, as an active ingredient, a biguanide antidiabetic drug selected from metformin, phenformin, and buformin, and is administered in combination with a cancer vaccine.
20. The agent for treating and/or preventing diseases associated with immune abnormalities according to Item 19, wherein the cancer vaccine is a cancer peptide vaccine (such as MAGE-3, MUC-1, WT1 peptide, P53, or NY-ESO1), a cancer protein vaccine, a dendritic cell vaccine, or a vaccine against a cancer proved to be developed by viral infection.
21. The agent for treating and/or preventing diseases associated with immune abnormalities according to any one of Items 13 to 20, wherein the diseases associated with immune abnormalities are selected from cancers, infections, and autoimmune diseases.
22. The agent for treating and/or preventing diseases associated with immune abnormalities according to Item 21, wherein the cancer is at least one cancer selected from head and neck cancers, esophagus cancer, gastric cancer, colorectal cancer, colon cancer, rectum cancer, liver cancer, gallbladder cancer, cholangiocarcinoma, biliary tract cancer, pancreatic cancer, lung cancer, breast cancer, ovarian cancer, cervical cancer, endometrial cancer, vaginal cancer, vulvar cancer, renal cancer, urothelial cancer, prostate cancer, testicular tumor, osteosarcoma, soft-tissue sarcoma, leukemia, myelodysplastic syndrome, malignant lymphoma, adult T-cell leukemia, multiple myeloma, skin cancer, brain tumor, pleural mesothelioma, and unknown primary cancer.
23. The agent for treating and/or preventing diseases associated with immune abnormalities according to Item 21 or 22, wherein the cancer treatment is suppression of cancer progression, and the cancer prevention is prevention of cancer recurrence.
24. An agent for suppressing progression, treating, preventing, and/or preventing recurrence of cancer, comprising, as an active ingredient, anti-PD-1 antibody, the agent being administered in combination with metformin, wherein the cancer is at least one cancer selected from head and neck cancers, esophagus cancer, gastric cancer, colorectal cancer, hepatocellular cancer, pancreatic cancer, non-small cell lung cancer, small cell lung cancer, breast cancer, ovarian cancer, cervical cancer, endometrial cancer, vaginal cancer, vulvar cancer, renal cell cancer, urothelial cancer, Hodgkin's lymphoma, follicular lymphoma, diffuse large B-cell lymphoma, multiple myeloma, malignant melanoma, glioblastoma and pleural mesothelioma.
25. The agent for treating and/or preventing diseases associated with immune abnormalities according to any one of Items 14 to 24, wherein the agent is used after a test using the test method of any one of Items 9 to 11.

A. A method for treating and/or preventing diseases associated with immune abnormalities, comprising using a therapeutic agent comprising, as an active ingredient, a biguanide antidiabetic drug selected from metformin, phenformin, and buformin, after a test using the test method according to any one of Items 9 to 11 was conducted.
B. The method for treating and/or preventing diseases associated with immune abnormalities according to Item A, wherein the diseases associated with immune abnormalities are selected from cancers, infections, and autoimmune diseases.
C. A method for treating and/or preventing diseases associated with immune abnormalities, comprising administering a therapeutic agent comprising, as an active ingredient, a biguanide antidiabetic drug selected from metformin, phenformin, and buformin, in combination with at least one therapeutic agent selected from immunosuppressive factor blocking agent and costimulatory receptor agonist.
D. A method for treating and/or preventing diseases associated with immune abnormalities, comprising administering a therapeutic agent comprising, as an active ingredient, at least one therapeutic agent selected from immunosuppressive factor blocking agent and costimulatory receptor agonist, in combination with a biguanide antidiabetic drug selected from metformin, phenformin, and buformin.
E. The method for treating and/or preventing diseases associated with immune abnormalities according to Item C or D, wherein the immunosuppressive factor blocking agent is one or any combination of antibodies or fusion proteins selected from anti-CTLA-4 antibody, anti-PD-1 antibody, anti-PD-L1 antibody, anti-PD-L2 antibody, PD-L1 fusion protein, PD-L2 fusion protein, anti-Tim-3 antibody, anti-LAG-3 antibody, anti-BTLA antibody, and anti-VISTA antibody.
F. The method for treating and/or preventing diseases associated with immune abnormalities according to Item E, wherein the anti-CTLA-4 antibody is Ipilimumab or Tremelimumab, the anti-PD-1 antibody is Nivolumab, Pembrolizumab, PDR-001, REGN-2810, BGB-A317, or AMP-514, and the anti-PD-L1 antibody is Atezolizumab, Avelumab, Durvalumab, or BMS-936559, and the PD-L2 fusion protein is AMP-224.
G. The method for treating and/or preventing diseases associated with immune abnormalities according to Item C or D, wherein the costimulatory receptor agonist is an antibody or any combination of antibodies selected from anti-CD137 antibody, anti-OX40 antibody, anti-HVEM antibody, anti-CD27 antibody, anti-GITR antibody, and anti-CD28 antibody.
H. A method for treating and/or preventing diseases associated with immune abnormalities, comprising administering a therapeutic agent comprising, as an active ingredient, a biguanide antidiabetic drug selected from metformin, phenformin, and buformin, in combination with a cancer vaccine.
I. The method for treating and/or preventing diseases associated with immune abnormalities according to Item H, wherein the cancer vaccine is a cancer peptide vaccine (such as MAGE-3, MUC-1, WT1 peptide, P53, or NY-ESO1), a cancer protein vaccine, a dendritic cell vaccine, or a vaccine against a cancer proved to be developed by viral infection.
J. A method for treating and/or preventing diseases accompanied by immune abnormalities, comprising administering immune cells that are enhanced in function by the method according to any one of Items 1 to 3.
K. The method for treating and/or preventing diseases associated with immune abnormalities according to any one of Items B to J, wherein the diseases associated with immune abnormalities are selected from cancers, infections, and autoimmune diseases.
L. The method for treating and/or preventing diseases associated with immune abnormalities according to Item K, wherein the cancer is at least one cancer selected from head and neck cancers, esophagus cancer, gastric cancer, colorectal cancer, colon cancer, rectum cancer, liver cancer (such as hepatocellular cancer), gallbladder cancer, cholangiocarcinoma, biliary tract cancer, pancreatic cancer, lung cancer (such as non-small cell lung cancer (squamous non-small cell lung cancer, non-squamous non-small cell lung cancer), or small-cell lung cancer), breast cancer, ovarian cancer, cervical cancer, endometrial cancer, vaginal cancer, vulvar cancer, renal cancer (such as renal cell cancer), urothelial cancer (such as bladder cancer or upper urinary tract cancer), prostate cancer, testicular tumor (such as germ cell neoplasm), osteosarcoma, soft-tissue sarcoma, leukemia, myelodysplastic syndrome, malignant lymphoma (such as Hodgkin's lymphoma, follicular lymphoma, or diffuse large B-cell lymphoma), adult T-cell leukemia, multiple myeloma, skin cancer (such as malignant melanoma or Merkel cell carcinoma), brain tumor (such as glioblastoma), pleural mesothelioma, and unknown primary cancer.
M. The method for treating and/or preventing diseases associated with immune abnormalities according to Item K or L, wherein the cancer treatment is suppression of cancer progression, and the cancer prevention is prevention of cancer recurrence.
N. A method for suppressing progression, treating, preventing, and/or preventing recurrence of cancer, comprising administering a therapeutic agent comprising, as an active ingredient, anti-PD-1 antibody (e.g., Nivolumab, Pembrolizumab), in combination with metformin, wherein the cancer is at least one cancer selected from head and neck cancers, esophagus cancer, gastric cancer, colorectal cancer, hepatocellular cancer, pancreatic cancer, non-small cell lung cancer, small cell lung cancer, breast cancer, ovarian cancer, cervical cancer, endometrial cancer, vaginal cancer, vulvar cancer, renal cell cancer, urothelial cancer, Hodgkin's lymphoma, follicular lymphoma, diffuse large B-cell lymphoma, multiple myeloma, malignant melanoma, glioblastoma, and pleural mesothelioma.
O. The method for treating and/or preventing diseases associated with immune abnormalities according to any one of Items C to N, wherein the method is used after a test using the test method of any one of Items 9 to 11.

### Advantageous Effects of Invention

The agent of the present invention for treating diseases associated with immune abnormalities is capable of improving immunity. For example, in cancer treatments, the agent of the present invention enables more effective treatment in combination with surgery, radial ray treatment, or chemotherapy, and thus is expected to improve the prognosis. Further, the agent of the present invention is expected to have a significant effect in the use as preoperative chemotherapy. Furthermore, the agent of the present invention is expected to have an effect of suppressing recurrence after treatment.

Further, the following effects can be assumed by the cell treatment using the method for enhancing immune cell function of the present invention. By treating self-derived cells with a biguanide antidiabetic drug selected from metformin, phenformin, and buformin ex vivo according to the method of the present invention, and then restoring the cells to the body from which the cells were obtained, it is possible to impart a high biophylactic ability against cancers or pathogenic microbes. By treating cells according to the method for enhancing immune cell function of the present invention, and then restoring the cells to the body from which the cells were obtained, effects of preventing cancers or infections can be expected.

### Brief Description of Drawings

Fig. 1 shows results of CD8⁺T cell multifunctionality analysis by using a flow cytometer. In peripheral blood mononuclear cells (PBMC) treated with PMA (Phorbol-12-Myristate-13-Acetate) /ionomycin, a gate was applied to CD8⁺T cells, and tablet cells were removed by FSC-A and FSC-H. Subsequently, a gate was applied to the lymphocyte regions of viable cells using parameters FSC-A and SSC-A, thereby analyzing expressions of PD-1 and Tim-3. Further, intracellular cytokines IFNγ, TNFα, and IL-2 were stained, thereby detecting multifunctionality (Example 1).
Fig. 2 shows results of detection of cells capable of simultaneously producing IFNγ, TNFα and IL-2 among CD8⁺T cells in the cells obtained by culturing PBMC of healthy subjects for an hour in the presence or absence of metformin, and then, after removing metformin, treating the cells with PMA/ionomycin (Example 1).
Fig. 3 shows results of analysis using peripheral blood mononuclear cells of cancer patients in a manner similar to that of Fig. 2 (Example 1).
Fig. 4 shows results of confirmation of expression frequency of exhaustion molecules PD-1 and Tim-3 in CD8⁺T cells obtained from healthy subjects (N=5) and cancer patients (N=5) (Example 2).
Fig. 5 shows procedures for confirming effects of a metformin treatment of cells ex vivo using mice (C57BL/6) (Example 5).
Fig. 6 shows results obtained by using a flow cytometer, showing cytokine production ability of T cells infiltrated into tumor cells in a test in which CD8⁺T cells derived from spleen cells of donor OT-I mice (TCR transgenic mice: TCR recognizes H-2Kb+OVA257-264, Cell vol.76, 17-27, 1994) treated with metformin ex vivo were introduced into a recipient C57BL/6 seven days after tumor cell transplantation (Example 5).
Fig. 7 shows results obtained by using a flow cytometer, showing Annexin V-positivity ratio (apoptosis ratio) of T cells infiltrated into tumor cells in a test in which CD8⁺T cells of donor OT-I mice treated with metformin ex vivo were introduced into a recipient C57BL/6 seven days after tumor cell transplantation (Example 5).
Fig. 8 shows results of measurement of tumor size in a test in which CD8⁺T cells of donor OT-I mice treated with metformin ex vivo were introduced into a recipient C57BL/6 seven days after tumor cell transplantation (Example 5).
Fig. 9 shows results of monitoring the tumor growth curve in a test in which CD8⁺T cells of donor OT-I mice treated with metformin (0, 10, 100 M) ex vivo were introduced into a recipient C57BL/6 seven days after tumor cell transplantation (Example 6).
Fig. 10 shows results of monitoring the tumor growth curve after free-water-drinking metformin administration and/or introduction of OVA vaccine (a fusion protein obtained by fusing OVA257-264 with the C terminus of mouse hsc70: Int. Immunol. 13, 1233-1242, 2001) into a recipient C57BL/6 seven days after tumor cell transplantation (Example 7).
Fig. 11 shows results of monitoring the tumor growth curve after free-water-drinking metformin administration and/or introduction of anti-PD-1 antibody (clone 4H2, a murinized chimeric antibody with a variable region of rat-derived anti-mouse PD-1 antibody and a constant region of mouse IgGκ1, provided by Ono Pharmaceutical Co., Ltd.) into a recipient C57BL/6 or BALB/c mice five days after tumor cell transplantation (MO5→C57BL/6, Meth A→BALB/c) (Example 8).
Fig. 12 shows a plot of tumor diameters of individual C57BL/6 mice (Example 8).
Fig. 13 shows a plot of tumor diameters of individual BALB/c mice (Example 8).

### Description of Embodiments

An embodiment of the present invention relates to a method for enhancing immune cell function, characterized by comprising treating removed immune cells with a therapeutic agent containing, as an active ingredient, a biguanide antidiabetic drug selected from metformin, phenformin and buformin in vitro; immune cells with enhanced immunity; and an agent for treating and/or preventing diseases associated with immune abnormalities containing, as an active ingredient, the immune cells with enhanced immunity.

Another embodiment of the present invention relates to multifunctionality of removed immune cells and to a method for evaluating multifunctionality of immune cells that are positive with respect to cytokine production ability for three kinds of cytokines, IL-2, TNFα, and IFNγ, with respect to a biguanide antidiabetic drug selected from metformin, phenformin, and buformin. Still another embodiment of the present invention relates to a method for evaluating sensitivity to immune cell therapeutic agent characterized by comprising using the immune cell multifunctionality evaluation method. When the multifunctionality of immune cells treated with a biguanide antidiabetic drug selected from metformin, phenformin, and buformin is determined to be significantly increased compared with the control, it can be evaluated that the sensitivity to immune cell therapeutic agent has been improved.

In this specification, "immune cells" means effector cells capable of interfering with target cells. The immune cells are particularly preferably CD8⁺T cells, but immune cells may also be selected from, for example, CD4⁺T cells, NK cells, γδT cells, NKT cells, B cells, and bone marrow cells. These cells encompass genetically modified cells such as iPS cells obtained from virus or cancer cell-specific CD8⁺T cells, or cells obtained by incorporating in naive CD8⁺T cells an antigen receptor (T cell antigen receptor: TCR) gene or a chimeric antibody (chimeric antigen receptor: CAR) gene that recognizes cancer cell surface expression molecules.

### 1. Method for Enhancing Immune Cell Function

In this specification, "immune cells with high immune function" means immune cells with a high content of cells that are positive with respect to cytokine production ability for three kinds of cytokines: IL-2, TNFα, and IFNγ.

The present invention relates to a method for enhancing immune cell function. More specifically, the method is performed by treating immune cells using a therapeutic agent containing, as an active ingredient, a biguanide antidiabetic drug selected from metformin, phenformin, and buformin. Further, the present invention also encompasses cells treated according to the method for enhancing immune cell function.

In this specification, immune cells enhanced in immunity by being treated with a therapeutic agent containing, as an active ingredient, a biguanide antidiabetic drug selected from metformin, phenformin, and buformin may also be referred to as "metformin-induced immune cells" (MTi cells). As explained above, the immune cells to be treated include all effector cells capable of interfering with target cells. For example, the immune cells to be treated may be selected from CD8⁺T cells, CD4⁺T cells, NK cells, γδt cells, NKT cells, B cells, and bone marrow cells. Examples of the cells mentioned above also encompass genetically modified cells such as iPS cells obtained from virus or cancer cell-specific CD8⁺T cells, and cells obtained by incorporating in naive CD8⁺T cells an antigen receptor (T cell antigen receptor: TCR) gene or a chimeric antibody (chimeric antigen receptor: CAR) gene that recognizes cancer cell surface expression molecules. The cells are particularly preferably CD8⁺T cells. The immune cells to be treated may be collected by methods known per se or any methods developed in the future. Further, it is not necessary to directly treat immune cells, for example, CD8⁺T cells. Insofar as immune cells, for example, CD8⁺T cells, are included in the cells, it is possible to treat peripheral blood mononuclear cells (PBMC) derived from peripheral blood obtain by an usual method.

PBMC may be obtained by methods known per se. For example, PBMC may be obtained by adding a whole blood sample from a donor to a Ficoll solution, and centrifuging the mixture, followed by gravity separation.

By culturing a medium in which a biguanide antidiabetic drug selected from metformin, phenformin, and buformin is added to PBMC for producing MTi cells, it is possible to enhance the function of immune cells.

More specifically, it is possible to enhance the function of CD8⁺T cells by culturing cells containing CD8⁺T cells such as PBMC at 37±0.5°C, 5% CO₂, for 3 to 12 hours, preferably 4 to 10 hours, most preferably 6 hours, using a medium containing a biguanide antidiabetic drug selected from metformin, phenformin, and buformin in an amount of about 1 to 500 µM, preferably 1 to 100 µM, more preferably about 5 to 100 µM, particularly preferably 10 to 100 µM per 10×10⁶/2 mL CD8⁺T cells. The medium is not particularly limited, and any medium capable of culturing CD8⁺T cells is sufficient. AIM-V^{(R)} medium (Invitrogen) is particularly preferable.

The present invention also encompasses immune cells enhanced in function by the above treatment. The immune cells with enhanced function mean an immune cell group with an increased content of cells that are positive with respect to cytokine production ability for three kinds of cytokines: IL-2, TNFα, and IFNγ. The obtained immune cells with enhanced function contain a large amount of CD8⁺T cells that become positive with respect to cytokine production ability for three kinds of cytokines after the cells are introduced into the body. The immune cells with enhanced function may be used as an agent for treating diseases associated with immune abnormalities.

Further, a cell treatment using the method for enhancing immune cell function of the present invention is assumed to have the following effects. By treating self-derived cells ex vivo with a biguanide antidiabetic drug selected from metformin, phenformin, and buformin according to the method of the present invention, and then restoring the resulting cells to the body from which the cells were obtained, it is possible to impart a high biophylactic ability against cancers or pathogenic microbes. The therapeutic agent containing, as an active ingredient, a biguanide antidiabetic drug selected from metformin, phenformin, and buformin is contraindicated for patients with liver dysfunction or kidney dysfunction, elderly people, patients who had lactic acidosis in the past, and the like. However, by treating self-derived cells according to the method for enhancing immune cell function ex vivo of the present invention, it is possible to eliminate concerns regarding side effects or safety in taking a biguanide antidiabetic drug. Further, in cancer immunotherapy, by performing an immune cell treatment in which immune cells enhanced in immunity according to the method of the present invention are placed back into the patient again, the ability to reach the target tumor region of the introduced cells can be improved compared with the case of taking a therapeutic agent containing a biguanide antidiabetic drug as an active ingredient; thus, improvement in the therapeutic effect can be expected. The method of the present invention may be used, for example, for preventing onset of cancer in healthy subjects. By proliferating lymphocytes obtained from peripheral blood of a healthy subject by a method known per se, cryopreserving and partially thawing the resulting cells, and then treating the cells using the method for enhancing immune cell function of the present invention and placing the cells back into the subject's body, it is possible to contribute to the prevention of diseases associated with immune abnormalities, such as cancers or infections.

Such a cell group with superior immunity may be conserved in a manner similar to that for general known cells for use in immunotherapy. The present invention also encompasses an agent for treating and/or preventing diseases associated with immune abnormalities containing, as an active ingredient, immune cells with enhanced immunity (MTi cells) prepared by the above method. The dosage, frequency, and interval in the administration of the agent for treating and/or preventing diseases associated with immune abnormalities are suitably determined according to, for example, the age, sex, weight, and symptoms of the patient.

In the diseases associated with immune abnormalities, T cell function is presumably exhausted, and therefore the immunity is presumably decreased or abnormally increased. Examples of such diseases include cancers, immunodeficiency diseases, autoimmune diseases, allergic diseases, and various refractory infections.

When the disease is cancer, examples of cancers include, but are not particularly limited to, head and neck cancers, esophagus cancer, gastric cancer, colon cancer, rectum cancer, liver cancer, gallbladder cancer, cholangiocarcinoma, biliary tract cancer, pancreatic cancer, lung cancer, breast cancer, ovarian cancer, cervical cancer, endometrial cancer, renal cancer, bladder cancer, prostate cancer, testicular tumor, osteosarcoma, soft-tissue sarcoma, leukemia, malignant lymphoma, multiple myeloma, skin cancer, brain tumor, and mesothelioma. Further, examples of immunodeficiency diseases include congenital immunodeficiency diseases and acquired immunodeficiency diseases. Examples of congenital immunodeficiency diseases include, but are not particularly limited to, severe combined immunodeficiency diseases, Wiskott-Aldrich syndrome, adenosine deaminase deficiency, and purine nucleotide/phosphorylase deficiency. Further, examples of acquired immunodeficiency diseases include, but are not particularly limited to, secondary immunodeficiencies caused by use of anticancer drugs, immunosuppressants, steroids, and the like, and AIDS caused by infection with human immunodeficiency virus. Examples of autoimmune diseases include, but are not particularly limited to, systemic lupus erythematosus, rheumatoid arthritis, Sjogren's syndrome, myasthenia gravis, pernicious anemia, and Hashimoto's thyroiditis. Examples of allergic diseases include, but are not particularly limited to, bronchitic asthma, cedar pollinosis, and hives. Examples of infections include viral infections and bacterial infections. Examples of viral infections include, but are not particularly limited to, digestive tract virus infections (such as enterovirus or cytomegalovirus), respiratory virus infections (infections with respiratory virus such as influenza virus, rhinovirus, coronavirus, parainfluenza virus, RS virus, adenovirus, or reovirus), herpes zoster caused by herpesvirus, diarrhea caused by rotavirus, viral hepatitis, and AIDS. Examples of bacterial infections include, but are not particularly limited to, infections with *Bacillus cereus, Vibrio parahaemolyticus,* enterohemorrhagic *E. coli, Staphylococcus aureus,* MRSA, *Salmonella, Botulinus,* and *Candida.*

### 2. Immune Cell Multifunctionality Evaluation Method

In this specification, the method for evaluating multifunctionality of removed immune cells is performed by evaluating the functions of cells having an ability to produce cytokines IL-2, TNFα and IFNγ in the removed immune cells. Evaluation of multifunctionality is performed with respect to cells obtained by treating immune cells with a therapeutic agent containing, as an active ingredient, a biguanide antidiabetic drug selected from metformin, phenformin, and buformin in vitro. The evaluation of cell function enables predicting the sensitivity of the cells to a biguanide antidiabetic drug selected from metformin, phenformin, and buformin, or the therapeutic effect. When the multifunctionality of immune cells treated with a biguanide antidiabetic drug selected from metformin, phenformin, and buformin is determined to be significantly increased compared with the control, it can be evaluated that the sensitivity of the immune cell to the therapeutic agent is improved. Thus, it is possible to evaluate the sensitivity of the removed immune cells to a biguanide antidiabetic drug selected from metformin, phenformin, and buformin. The functions of exhausted CD8⁺T cells or CD8⁺T cells with an exhaustion tendency are recovered by metformin. Normal CD8⁺T cells (i.e., PBMC from healthy subjects) are less sensitive to metformin because of their normality, and therefore a result with a decrease in multifunctionality by a metformin treatment is more often observed. In contrast, since cancer patients with many exhausted CD8⁺T cells are highly sensitive to metformin, a result with an increase or no change in multifunctionality by a metformin treatment is presumably more often observed.

For the immune cell multifunctionality evaluation method of the present invention, it is preferable to stimulate the removed immune cells, such as PBMC, because it is necessary to cause intracellular activation and facilitate cytokine production. The cell stimulation may be performed by methods known per se, such as stimulation using a protein kinase activator (highly potent protein kinase C (PKC) activator: PMA) and ionomycin. The stimulation using PMA and ionomycin is performed using, for example, 20 to 100 ng/mL, preferably 30 to 80 ng/mL, more preferably 50 ng/mL PMA, and 1 to 10 µM, preferably 1 to 5 µM, more preferably 2 µM ionomycin. The stimulation using PMA and ionomycin may be performed by culturing PBMC at 37±0.5°C for 3 to 12 hours, preferably 4 to 10 hours, most preferably 6 hours, using a medium containing PMA and ionomycin at a concentration selected from the above range. In this case, to block the release of produced cytokine to the outside of the cells, it is preferable to use a therapeutic agent that blocks protein transport from the Golgi apparatus in the cytoplasm, such as monensin, brefeldin A, or the like.

The evaluation of immune cell multifunctionality of the present invention is characterized by confirming cells with a production ability with respect to cytokines IL-2, TNFα and IFNγ in CD8⁺T cells included in immune cells, and measuring the ratio (cell-positivity ratio) of (B) cells that are positive with respect to the cytokine production ability for three kinds of cytokines to (A) the population, i.e., the CD8⁺T cell group. In this specification, the cell-positivity ratio may be calculated according to the formula below. The cell-positivity ratio may also be calculated according to the analysis results obtained with a measurement device such as a flow cytometer. Cell-positivity ratio (%) = (number of cells in B)/(number of cells in A)×100

In this specification, "(B) cells that are positive with respect to cytokine production ability for three kinds of cytokines" means CD8⁺T cells that are determined to be positive with respect to production ability for all of the three cytokines IL-2, TNFα and IFNγ at the same time when the production ability of these cytokines is individually measured. More specifically, "(B) cells that are positive with respect to cytokine production ability for three kinds of cytokines" means cells that are determined to be positive in a gating with respect to one of the three cytokines, and then determined to be positive also with respect to the remaining two cytokines.

The immune cell multifunctionality evaluation of the present invention more specifically relates to a CD8⁺T cell function evaluation method comprising steps 1) to 4) below.
1) a step of treating removed immune cells in vitro with a therapeutic agent containing, as an active ingredient, a biguanide antidiabetic drug selected from metformin, phenformin, and buformin;
2) a step of stimulating the treated immune cells using PMA and ionomycin after the cells are centrifuged and washed;
3) a step of applying a gate to CD8⁺T cells in the immune cells stimulated in step 2) using a flow cytometer analysis; and
4) a step of measuring cytokine production ability for three kinds of cytokines, IL-2, TNFα, and IFNγ, of the gated CD8⁺T cells.

In the CD8⁺T cell function evaluation method of the present invention, expressions of Tim-3 and/or PD-1 may also be measured.

Further, more specifically, the measurement may also be performed as follows.

Peripheral blood is obtained from a test subject, and PBMC is separated using a lymphocyte-separating reagent and is stored in a test tube. The obtained PBMC may be cryopreserved until immediately before the test. The test cells (for example, PBMC) thawed before the test may be cultured at 37±0.5°C, 5% CO₂, for 1 to 12 hours, preferably 4 to 10 hours, most preferably 6 hours using a medium containing a biguanide antidiabetic drug selected from metformin, phenformin, and buformin, particularly preferably metformin, in an amount of 1 to 500 µM, preferably 1 to 100 µM, more preferably about 5 to 100 µM, particularly preferably about 10 to 100 µM. The medium is not particularly limited and any medium capable of culturing test cells is sufficient. AIM-V^{(R)} medium (Invitrogen) is particularly preferable. It is preferable that the cells are washed with medium or the like, and are cultured in the presence of a cell activation reagent (potent nanomolar activator of protein kinase C (PMA, 50 ng/mL) and ionomycin (1 µM)), thereby giving nonspecific stimulation to T cells. Thereafter, it is preferable that the cells are treated with an intracellular protein transport inhibitor to keep the produced cytokines inside the cells. The PBMC thus treated is isolated and washed with a cell-staining buffer 1 to 4 times; after a staining buffer is added, the cells may be cultured at 2 to 10°C, preferably at 3 to 5°C, and most preferably 4±1°C. The cells are washed with a cell-washing buffer, and staining of cytokines IL-2, TNFα, and IFNγ is performed. The cells are isolated, washed, and analyzed using FACS, and values regarding the production amounts of three kinds of cytokines, IL-2, TNFα, and IFNγ, are compared between a control untreated with a biguanide antidiabetic drug and the cells treated with a biguanide antidiabetic drug.

After a gate is applied to CD8⁺T cells in the flow cytometer analysis of the present invention, the tablet cells are removed by FSC-A and FSC-H of FSC (forward scatter). Then, a gate is applied to the lymphocyte group using parameters FSC-A and SSC-A, thereby analyzing the expressions of Tim-3 and/or PD-1. Further, it is possible to stain the intracellular cytokines IL-2, TNFα and IFNγ, thereby detecting the multifunctionality. In this specification, an immune cell ability to simultaneously produce intracellular cytokines IL-2, TNFα and IFNγ may occasionally be referred to as "multifunctionality." Regarding the multifunctionality, the strongest effector cell is a cell capable of simultaneously producing three kinds of cytokine, and the second strongest effector cell is a cell capable of simultaneously producing two kinds of cytokine. A cell producing only one kind of cytokine is not regarded as having multifunctionality, because the effector function of this cell is limited.

The present invention also encompasses a method for testing diseases associated with immune abnormalities using the immune cell multifunctionality evaluation method of the present invention. In this specification, a test of a disease associated with immune abnormalities means a test supplementarily used for predicting efficacy of a therapeutic agent against the disease, predicting severity of the disease, predicting prognosis of the treatment of the diseases, or predicting onset of the disease. Cancer patients who are not responsive to metformin at all may be regarded as having severe immune exhaustion. The immune cell multifunctionality evaluation of the present invention may be supplementarily used for judging prediction of efficacy of a therapeutic agent against the disease, predicting severity of the disease, predicting prognosis, or predicting onset of the disease, with regard to diseases associated with immune abnormalities.

In recent years, the mechanism of immune exhaustion has been clarified, and there are reports that inhibition of exhaustion molecules is important in cancer treatment. The test method of the present invention enables combined use of detection of a cell group simultaneously producing three kinds of cytokines and detection of exhaustion molecules, thereby easily detecting the immune status of immune cells, i.e., CD8⁺T cells, of the patients. If the cells are expressing exhaustion molecules such as PD-1 or Tim-3, the cells are determined to be exhausted cells. However, if the multifunctionality of the cell group is recovered by a metformin treatment, it can be judged that the exhaustion is undoubtedly canceled (immunity is recovered).

In previous cancer vaccine treatments, the patients to be subjected to the treatments were selected based on the presence or absence of vaccine antigen expression in the cancer tissues. However, the evaluation method of the present invention enables detection of the immune status before the immunotherapy, thereby greatly contributing to the selection of treatment, in addition to the antigen expression.

In this specification, "therapeutic agent" in the "prediction of efficacy of a therapeutic agent" refers to a therapeutic agent containing, as an active ingredient, a biguanide antidiabetic drug selected from metformin, phenformin, and buformin. The treatment effects with respect to diseases associated with immune abnormalities are superior in a donor of removed immune cells that were treated by a therapeutic agent containing, as an active ingredient, a biguanide antidiabetic drug selected from metformin, phenformin, and buformin in vitro and approved in terms of multifunctionality of intracellular cytokines IL-2, TNFα, and IFNγ by a flow cytometer analysis.

By applying an agent for treating and/or preventing diseases associated with immune abnormalities containing, as an active ingredient, a biguanide antidiabetic drug selected from metformin, phenformin, and buformin to healthy subjects, it is possible to impart potential multifunctionality to CD8⁺T cells. As a result, it is possible to expect an effect of alleviating an immune compromise symptom (chronic refractory infection, cancer patients, diabetes patients, and the like) or, for example, the following conditions. For example, the conditions include consumption of physical strength, decrease in physical strength due to aging, immunodeficiencies such as combined immunodeficiency diseases, antibody production disorder, or complement deficiency, hypothyroidism, immune compromise due to radiation, congenital immunodeficiency diseases, acquired immunodeficiency diseases (including AIDS). It is assumed that, by administering an agent for treating and/or preventing diseases associated with immune abnormalities, the immunity is recovered, and the general immune system is recovered, thereby leading to alleviation of the conditions and symptoms, or remission.

The present invention also encompasses an agent for treating and/or preventing diseases associated with immune abnormalities containing, as an active ingredient, a biguanide antidiabetic drug selected from metformin, phenformin, and buformin. The active ingredient contained in the agent for treating and/or preventing diseases associated with immune abnormalities of the present invention is capable of recovering the function of immune cells and suppressing apoptosis, thereby recovering immunity. The recovery of immune cell function means, in the case of CD8⁺T cells, recovery of an ability to simultaneously produce three kinds of cytokines: IL-2, TNFα, and IFNγ. Further, in the case of other immunocompetent cells, the recovery of immune cell function means recovery of the function inherent in the cell due to suppression of apoptosis. For example, the recovery of immune cell function presumably means an increase in IFNγ production ability in the case of CD4T cells, NK cells, NKT cells, and γδT cells; an increase in antibody production ability in the case of B cells; and an increase in differentiation induction ability in the case of bone marrow cells.

The biguanide antidiabetic drug selected from metformin, phenformin, and buformin serving as the active ingredient of the agent for treating and/or preventing diseases associated with immune abnormalities of the present invention may be administered in an amount of 1 to 5000 mg, preferably 10 to 4000 mg, more preferably about 50 to 3000 mg, particularly preferably about 100 to 2500 mg per day for an adult. The active ingredient may be administered once a day, or may be divided into 2 to 4 doses a day. Further, as necessary, the active ingredient may be administered for several days. The interval and frequency of administration may be suitably determined.

### 3. Combined Use with Other Therapeutic Agents

In the treatment and/or prevention of diseases associated with immune abnormalities of the present invention, when a biguanide antidiabetic drug selected from metformin, phenformin, and buformin is administered to a patient with these diseases, the agent may be combined with other therapeutic agents. In the case of separately administering the biguanide antidiabetic drug of the present invention and other therapeutic agents, it is possible to administer the biguanide antidiabetic drug of the present invention first, and then administer other therapeutic agents; or administer other therapeutic agents first, and then administer the biguanide antidiabetic drug of the present invention. Further, during the administration period, it is possible to simultaneously administer all of the therapeutic agents for a limited certain period of time. Further, the therapeutic agents may be administered by the same method or different methods. Depending on the property of the therapeutic agent, a kit including a preparation containing the biguanide antidiabetic drug of the present invention and other therapeutic agents may be provided. The biguanide antidiabetic drug of the present invention may be suitably selected based on the dosages mentioned above. The dosages of other therapeutic agents may also be suitably selected based on the dosages clinically used. The other therapeutic agents encompass agents discovered in the past and agents discovered in the future. Major examples of other therapeutic agents include immunosuppressive factor blocking agents. In cancer cells or cancer microenvironments, various immunosuppressive factors that interfere with immune response to the cancer are present. There are various immune checkpoints in the process of immune response. In particular, the functions of negative costimulatory molecules such as CTLA-4 or PD-1 are important checkpoints for the control of autologous reactivity. Examples of such molecules that inhibit immune checkpoints, i.e., immunosuppressive factor blocking agents, include anti-CTLA-4 antibody (for example, Ipilimumab, Tremelimumab), anti-PD-1 antibody (for example, human anti-human PD-1 monoclonal (neutralizing) antibody (for example, Nivolumab, REGN-2810), humanized anti-human PD-1 monoclonal (neutralizing) antibody (for example, Pembrolizumab, PDR-001, BGB-A317, AMP-514 (MEDI0680))), anti-PD-L1 antibody (for example, Atezolizumab (RG7446, MPDL3280A), Avelumab (PF-06834635, MSB0010718C), Durvalumab (MEDI4736), BMS-936559), anti-PD-L2 antibody, PD-L1 fusion protein, PD-L2 fusion protein (for example, AMP-224), anti-Tim-3 antibody (for example, MBG453), anti-LAG-3 antibody (for example, BMS-986016, LAG525), anti-KIR antibody (for example, Lirilumab), anti-BTLA antibody, and anti-VISTA antibody. Further, examples of other therapeutic agents also include costimulatory receptor agonists such as anti-CD137 antibody (for example, Urelumab), anti-OX40 antibody (for example, MEDI6469), anti-HVEM antibody, anti-CD27 antibody (for example, Varlilumab), anti-GITR antibody (for example, MK-4166) and anti-CD28 antibody. Any one or more kinds of antibodies or fusion proteins of these immunosuppressive factor blocking agents and costimulatory receptor agonists may be combined with the biguanide antidiabetic drug of the present invention. Examples of diseases that are expected to be treated or prevented by a combination of the biguanide antidiabetic drug selected from metformin, phenformin, and buformin of the present invention, and an immunosuppressive factor blocking agent and/or a costimulatory receptor agonist include cancer (such as, but are not particularly limited to, head and neck cancers, esophagus cancer, gastric cancer, colorectal cancer, colon cancer, rectum cancer, liver cancer (such as hepatocellular cancer), gallbladder cancer, cholangiocarcinoma, biliary tract cancer, pancreatic cancer, lung cancer (such as non-small cell lung cancer (squamous non-small cell lung cancer, non-squamous non-small cell lung cancer), or small-cell lung cancer), breast cancer, ovarian cancer, cervical cancer, endometrial cancer, vaginal cancer, vulvar cancer, renal cancer (such as renal cell cancer), urothelial cancer (such as bladder cancer or upper urinary tract cancer), prostate cancer, testicular tumor (such as germ cell neoplasm), osteosarcoma, soft-tissue sarcoma, leukemia, myelodysplastic syndrome, malignant lymphoma (such as Hodgkin's lymphoma, follicular lymphoma, or diffuse large B-cell lymphoma), adult T-cell leukemia, multiple myeloma, skin cancer (such as malignant melanoma or Merkel cell carcinoma), brain tumor (such as glioblastoma), pleural mesothelioma, and unknown primary cancer. Among these cancers, it is possible to maximally exert the antitumor effects for, in particular, cancer patients or cancer types receiving insufficient therapeutic effects from a sole use of immunosuppressive factor blocking agent or costimulatory receptor agonist.

The agent for treating and/or preventing diseases associated with immune abnormalities of the present invention preferably has, for example, a liquid form when it is intravenously administered. The liquid can be prepared, for example, by using solvents such as purified water, physiological saline solution, alcohols including ethanol, propylene glycol, glycerin, and polyethylene glycol, or triacetin. Adjuvants such as antiseptics, wetting agents, emulsifiers, dispersants, stabilizers, and the like may also be added to the preparation. Further, the preparation may be administered as a suspension.

Further, solid preparations such as tablets, pills, powdered drugs, granules, or fine granules may be prepared, for example, by a standard method by adding carriers such as sodium bicarbonate, calcium carbonate, starch, sucrose, mannitol, or carboxymethylcellulose, and additives such as calcium stearate, magnesium stearate, or glycerin. Further, the agent may be prepared into an enteric-coated preparation by performing enteric coating by spraying an enteric coating substance such as organic solvent or aqueous solution of cellulose acetate phthalate, hydroxypropylmethylcellulose phthalate, polyvinyl alcohol phthalate, styrene-maleic anhydride copolymer, methacrylic acid-methyl methacrylate copolymer, or the like. Examples of pharmaceutically acceptable carriers include other general adjuvants, fragrances, and stabilizers or antiseptics.

Further, the present invention also encompasses an agent and a method for treating and/or preventing diseases associated with immune abnormalities by administering a combination of a therapeutic agent containing, as an active ingredient, a biguanide antidiabetic drug selected from metformin, phenformin, and buformin with a cancer vaccine. "Cancer vaccine" includes cancer peptide vaccines (MAGE-3, MUC-1, WT1 peptide, P53, NY-ESO1, etc.), cancer protein vaccines, dendritic cell vaccines, gene therapy vaccines, and the like. Examples of dendritic cell vaccines include dendritic cell vaccines in which cancer peptide or cancer antigen protein is captured, dendritic cell vaccines in which cancer cell derived mRNA is transduced (and serve also as a gene therapy vaccine), and dendritic cell vaccines cultured with cancer antigen protein, such as prostatic acid phosphatase and GM-CSF fusion protein (such as Provenge). Examples of cancer vaccines include vaccines against cancers proved to be developed by viral infections, such as hepatitis B vaccine derived from hepatitis B virus, or cervical cancer prophylactic vaccine derived from human papilloma virus. By combining the agent for treating and/or preventing diseases associated with immune abnormalities of the present invention with various vaccines, it is possible to greatly enhance the vaccine effects. As a result, reduction in dosages of cancer vaccines and the like can be expected, thereby reducing side effects caused by vaccines and adjuvants.

The present invention also encompasses a method for treating and/or preventing diseases associated with immune abnormalities comprising using an agent for treating and/or preventing diseases associated with immune abnormalities.

### Examples

For further understanding of the present invention, the present invention is more specifically described below with reference to Reference Examples and Examples. However, the present invention is not limited to these examples. The research using clinical samples below is approved by the ethics committee of Okayama University. The Examples below show case analysis of cancers at different stages using peripheral blood of stage I primary lung cancer patients and metastatic lung cancer (i.e., stage IV) patients. The cancer stage is in accordance with the 7th edition of the UICC TNM Classification Staging Matrix (lung).

### Example 1: Immune Cell Multifunctionality Evaluation Method

This Example describes a method for evaluating immunity of immune cells. First, immune cell multifunctionality was analyzed using a flow cytometer. Subsequently, changes in multifunctionality of immune cells by a metformin treatment were confirmed in healthy subjects and cancer patients. The treatment of immune cells was performed using the (1) materials and (2) method below.

### (1) Materials

▪ Lymphocyte-separating reagent: Ficoll-Paque^{(R)} (GE Healthcare)
▪ Cell cryopreservation liquid: Bambang Car (Nippon Genetics)
▪ Human cell medium: AIM V^{(R)} Medium (Gibco)
▪ Cell activation reagent:
   Potent nanomolar activator of protein kinase C (Sigma-Aldrich) Ionomycin (Sigma-Aldrich)
▪ Intracellular protein transport inhibitor: BD Gorgi Stop™ (containing monensin)(BD Biosciences)
▪ Staining buffer: 0.58 g of EDTA (Nacalai Tasque) dissolved in 1 L of PBS (Gibco) containing 2% FCS (Thermo)
▪ Cell fixing solution/Permeation buffer
BD Cytofix/Cytoperm™ (BD Biosciences)
BD Perm/Wash™ (dilute 1:10 in distilled H₂O prior to use) (BD Biosciences)

### (2) Method

1. Peripheral blood was obtained from a test subject, and PBMC was separated by a density-gradient centrifugation using a lymphocyte-separating reagent Ficoll-Paque^{(R)} (GE Healthcare). 3 mL of Ficoll-Paque^{(R)} was dispensed in a 15-mL tube. 8 mL of the peripheral blood was gently placed on the reagent. At this time, care should be taken not to mix Ficoll-Paque^{(R)} with the peripheral blood. Centrifugation was performed at 400 g for 30 minutes, thereby separating PBMC.
2. The separated PBMC was preserved in a -150°C freezer or liquid nitrogen using Bambang Car cell preservation liquid by adjusting the cell number to 2.0×10⁶ cell/tube.
3. In the test, the thawed PBMC was cultured for 1 to 10 hours in the presence or absence of 10 µM metformin. The culture was performed using a 24-well plate, and the cell number was adjusted to about 5.0×10⁵ cell/well.
4. The cells were collected after 1 to 10 hours, washed with medium, and cultured in the presence of a cell activation reagent (potent nanomolar activator of protein kinase C (PMA, 50 ng/mL), ionomycin (1 µM)) for 6 hours, thereby non-specifically stimulating T cells. At this time, BD Gorgi Stop^{™} was added to keep the produced cytokines inside the cells.
5. The cells were isolated and washed twice with a cell-staining buffer. First, staining of CD8, PD-1, Tim-3, which are molecules expressed on the T cell surface, was performed. In the staining, a staining buffer was added in an amount of 50 µL per sample. The culture was performed at 4°C for 30 minutes.
6. The cells were collected and washed twice with a cell-staining buffer. 500 µL of BD Cytofix/Cytoperm^{™} was added and cultured at 4°C for 30 minutes.
7. The cells were collected and washed twice with BD Perm/Wash^{™}. Staining of cytokines IL-2, TNFα, and IFNγ was performed.
   In the staining, BD Perm/Wash^{™} was added in an amount of 50 µL per sample. The culture was performed at 4°C for 30 minutes.
8. The cells were collected and washed twice with BD Perm/Wash^{™}, and 200 µL of BD Perm/Wash^{™} was added.
9. Analysis was performed using a FACSCanto^{™} II flow cytometer (Becton, Dickinson and Company).

### (3) Analysis of Immune Cell Multifunctionality Using a Flow Cytometer

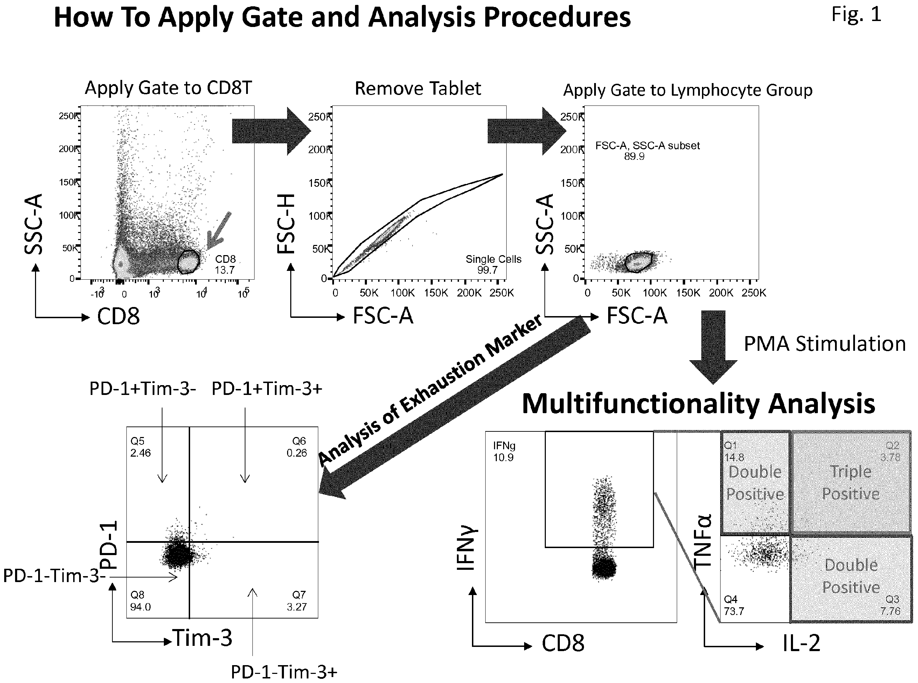

Among the PBMC treated with PMA/ionomycin in the method shown in (2) above, first, a gate was applied to CD8⁺T cells using a FACSCanto^{™} II flow cytometer (Becton, Dickinson and Company), and then tablet cells were removed by FSC-A and FSC-H. Using parameters FSC-A and SSC-A, a gate was applied to a lymphocyte group, thereby analyzing expression of PD-1 and Tim-3. Further, intracellular cytokines IFNγ, TNFα, and IL-2 were stained, and multifunctionality of CD8⁺T cells contained in the PBMC was analyzed (Fig. 1).

### (4) Analysis of Multifunctionality of CD8⁺T Cells of Healthy Subjects

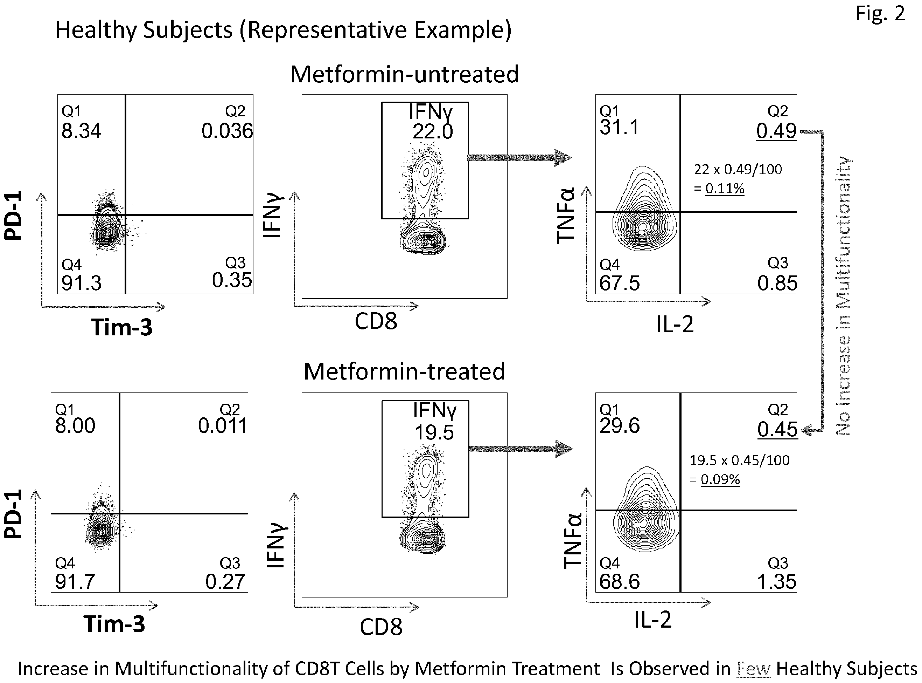

PBMC obtained from healthy subjects (N=10) was cultured for an hour in the presence or absence of metformin by the method shown in (2). After metformin was removed, the PBMC was subjected to stimulating culture with PMA/ionomycin for 6 hours by the method shown in (3). Among the resulting PBMC, CD8⁺T cells capable of simultaneously producing three kinds of cytokines, i.e., IFNγ, TNFα, and IL-2, were detected. The CD8⁺T cells untreated with metformin were determined to be 0.11%, and the CD8⁺T cells treated with metformin were determined to be 0.09% (Fig. 2). Accordingly, it was judged that multifunctionality of CD8⁺T cells was not increased by a metformin treatment in healthy subjects.

In the PBMC of healthy subjects treated as above, the ratios of CD8⁺T cells producing three kinds of cytokines (IFNγ/TNFα/IL-2), CD8⁺T cells producing two kinds of cytokines (IFNγ/TNFα or IFNγ/IL-2), and CD8⁺T cells producing one kind of cytokine (IFNγ) to the entire CD8⁺T cells were calculated and shown in Table 1. The calculation results were compared between the metformin-untreated group (control) and the metformin-treated group (metformin). Regarding the ratios of the cytokine-producing CD8⁺T cells, a 10% or more increase by a metformin treatment is denoted by an upward arrow (↑), a 10% or more decrease by a metformin treatment is denoted by a downward arrow (↓), and other cases with no significant differences is denoted by a rightward arrow (→).

**Table 1**

| ID | IFNγ/TNFα/IL-2 | | | IFNγ/TNFα | | | IFNγ/IL-2 | | | IFNγ | | |
|---|---|---|---|---|---|---|---|---|---|---|---|---|
| | control | metformin | | control | metformin | | control | metformin | | control | metformin | |
| 1 | 0.15 | 0.03 | ↓ | 1.53 | 1.50 | → | 0.11 | 0.03 | ↓ | 1.75 | 1.68 | → |
| 2 | 0.92 | 0.48 | ↓ | 15.16 | 11.17 | ↓ | 0.66 | 0.21 | ↓ | 5.55 | 6.23 | ↑ |
| 3 | 0.01 | 0.00 | → | 0.17 | 0.12 | ↓ | 0.06 | 0.05 | ↓ | 0.75 | 0.95 | ↑ |
| 4 | 0.04 | 0.01 | ↓ | 0.98 | 1.08 | ↑ | 0.11 | 0.14 | ↑ | 2.37 | 1.87 | ↓ |
| 6 | 0.14 | 0.09 | ↓ | 4.20 | 3.98 | → | 0.29 | 0.24 | ↓ | 10.87 | 12.00 | ↑ |
| 8 | 3.91 | 3.79 | → | 35.44 | 31.38 | ↓ | 0.55 | 0.47 | ↓ | 13.80 | 13.18 | → |
| 10 | 0.12 | 0.09 | ↓ | 4.19 | 3.54 | ↓ | 0.26 | 0.48 | ↑ | 18.34 | 16.70 | → |
| 12 | 2.22 | 2.16 | → | 25.78 | 32.26 | ↑ | 0.64 | 0.71 | ↑ | 7.25 | 8.18 | ↑ |
| 16 | 0.80 | 0.37 | ↓ | 19.64 | 11.52 | ↓ | 1.04 | 0.28 | ↓ | 21.13 | 11.64 | ↓ |
| 18 | 0.61 | 0.48 | ↓ | 14.91 | 16.84 | ↑ | 0.15 | 0.15 | → | 4.73 | 4.62 | → |

### (5) Analysis of Multifunctionality of CD8⁺T Cells in Cancer Patients

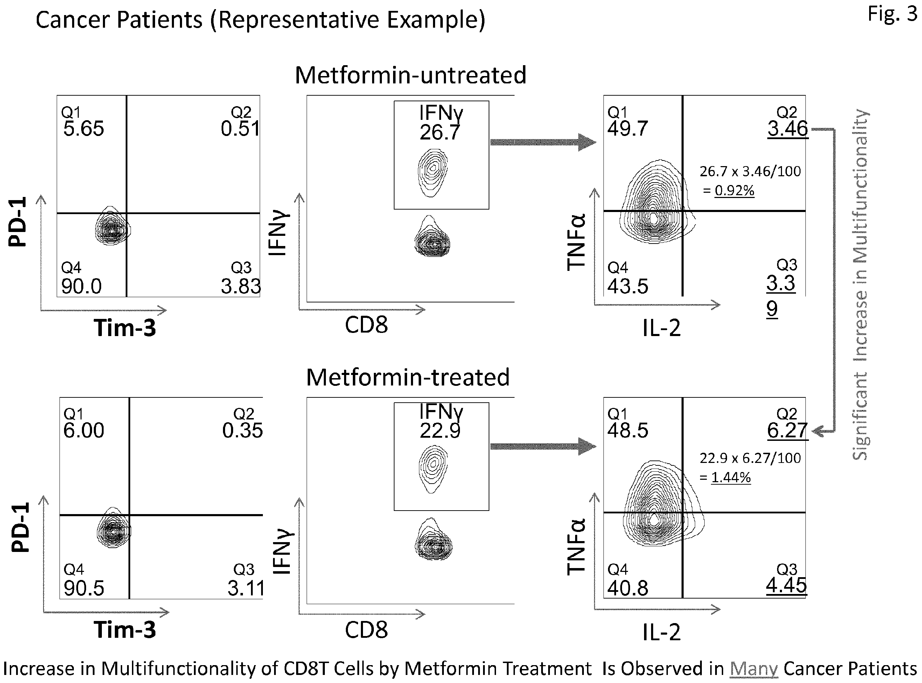

Each PBMC obtained from 31 primary lung cancer patients (stage I) was cultured for an hour in the same manner as in (4) in the presence or absence of metformin. After metformin was removed, each resulting PBMC was analyzed. In a representative example, the CD8⁺T cells untreated with metformin were determined to be 0.92%, and the CD8⁺T cells treated with metformin were determined to be 1.44% (Fig. 3). Accordingly, it was judged that, in comparison with healthy subjects, multifunctionality of CD8⁺T cells was increased by a metformin treatment in many cancer patients.

Among the cancer patients treated as above, the ratios of CD8⁺T cells producing three kinds of cytokines (IFNγ/TNFα/IL-2), CD8⁺T cells producing two kinds of cytokines (IFNγ/TNFα or IFNγ/IL-2), CD8⁺T cells producing one kind of cytokine (IFNγ) with respect to the entire CD8⁺T cells were calculated according to the same analysis as in (4), and shown in Table 2. The calculation results were compared between the metformin-untreated group (control) and the metformin-treated group (metformin). Regarding the ratios of the cytokine-producing CD8⁺T cells, a 10% or more increase by a metformin treatment is denoted by an upward arrow (↑), a 10% or more decrease by a metformin treatment is denoted by a downward arrow (↓), and other cases with no significant differences is denoted by a rightward arrow (→).

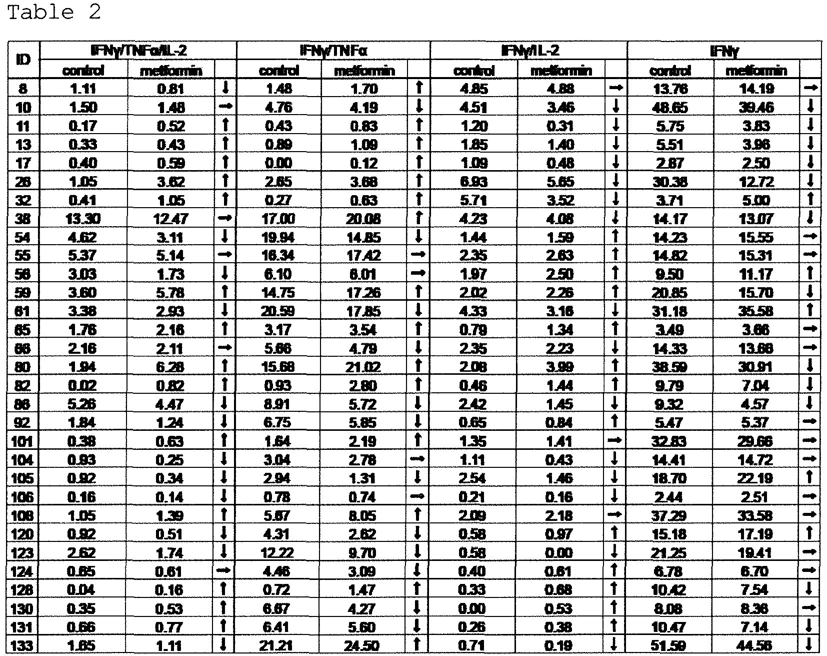

### Example 2: Immune Cell Multifunctionality Evaluation Method

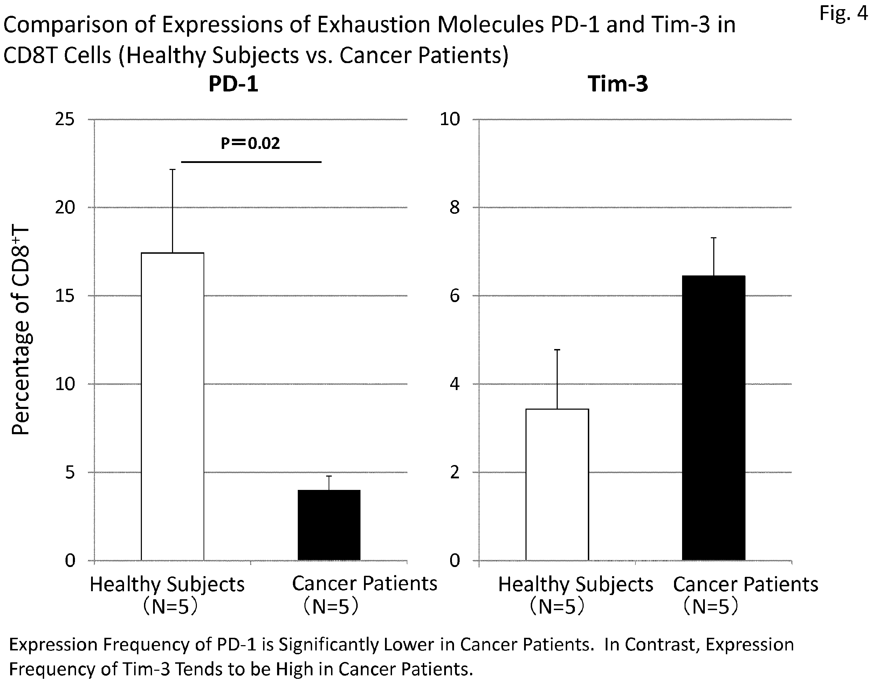

This Example examined expression frequencies of exhaustion molecules PD-1 and Tim-3 in CD8⁺T cells of five healthy subjects and five primary lung cancer patients (stage I). It was revealed that the expression of PD-1 was significantly low in cancer patients. Further, although there was a tendency that the expression of Tim-3 was high in cancer patients, no significant difference was observed with this sample number (Fig. 4). However, since healthy subjects and cancer patients clearly have different expression patterns in CD8⁺T cells, more detailed multifunctionality analysis becomes possible by combining expression patterns and multifunctionality analysis of PD-1 and Tim-3, in addition to the total CD8⁺T cells.

### Example 3: Test Results of Multifunctionality Recovery by metformin

This Example analyzed test results of recovery of multifunctionality of CD8⁺T cells by metformin in 10 healthy subjects and 31 cancer patients (stage I) based on the results obtained by the method in Example 1. The analysis results are shown in Table 3 below. Table 3 shows the results of cytokine production abilities of four kinds of CD8⁺T cells, i.e., PD-1-positive, Tim-3-negative CD8⁺T cells (PD-1+, Tim-3-), PD-1-positive, Tim-3-positive CD8⁺T cells (PD-1+, Tim-3+), PD-1-negative, Tim-3-positive CD8⁺T cells (PD-1-, Tim-3+), and PD-1-negative, Tim-3-negative CD8⁺T cells (PD-1-, Tim-3-), in addition to the total CD8⁺T cells (total CD8T). Cytokine production ability refers to cytokine production ratios of (A) three kinds of cytokines, IFNγ/TNFα/IL-2, (B) two kinds of cytokines, IFNγ/TNFα, (C) two kinds of cytokines, FNγ/IL-2, and (D) one kind of cytokine, IFNγ.

The results shown in Table 3 revealed that cytokine production ability for three kinds of cytokines were significantly different between healthy subjects and cancer patients in the total CD8⁺T cells, PD-1-negative, Tim-3-positive CD8⁺T cells, PD-1-negative, and Tim-3-negative CD8⁺T cells. In particular, in the total CD8⁺T cells, in healthy subjects, the increase was 0/10 (0%), and in cancer patients, the increase was 14/31 (45.2%). It was thus clarified that increase in multifunctionality by a metformin treatment more easily occurred in cancer patients. Further, ratios of decrease in multifunctionality by a metformin treatment were 7/10 (70%) in healthy subjects, and 12/31 (38.7%) in cancer patients. This shows a clear decrease in multifunctionality in healthy subjects. Regarding the cytokine production ability for two kinds of cytokines, there was a clear difference between healthy subjects and cancer patients in the total CD8⁺T cells, or PD-1-negative, Tim-3-positive CD8⁺T cells. Regarding the cytokine production ability for 1 kind of cytokine (in this case, IFNγ), there was no clear difference between healthy subjects and cancer patients.

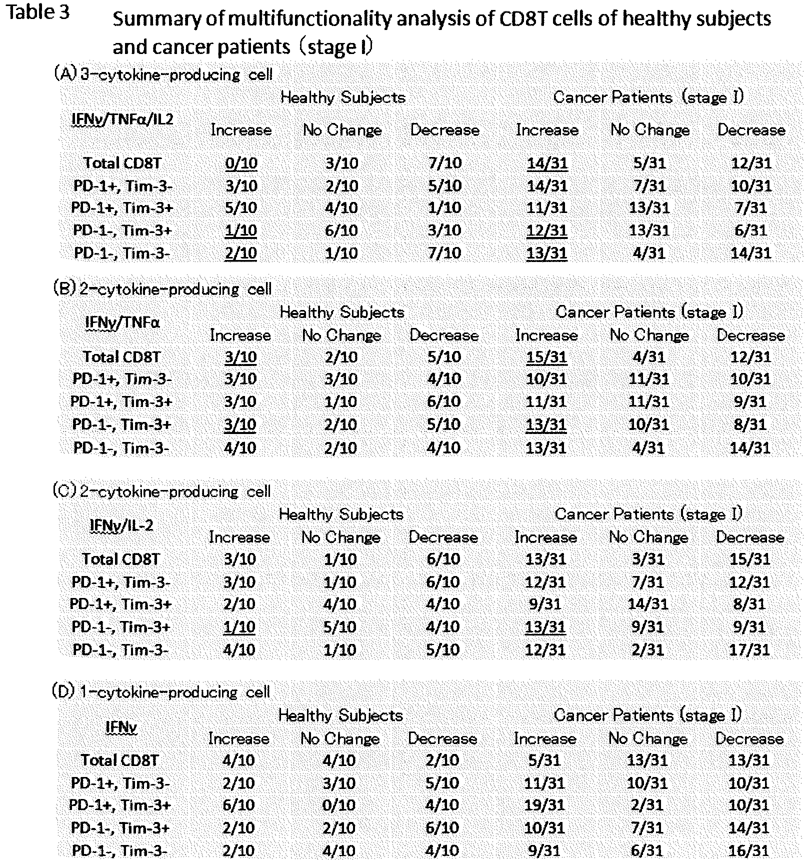

### Example 4: Immune Cell Multifunctionality Evaluation Method

This Example analyzed test results regarding recovery of multifunctionality of CD8⁺T cells by metformin in 10 healthy subjects and five metastatic lung cancer patients (stage IV) by the same method as in Example 1. The analysis results are shown in Table 4 below. As in Table 3, in Table 3, in addition to the total CD8⁺T cells (total CD8T), cytokine production abilities were confirmed in four kinds of CD8⁺T cells, i.e., PD-1-positive, Tim-3-negative CD8⁺T cells, PD-1-positive, Tim-3-positive CD8⁺T cells, PD-1-negative, Tim-3-positive CD8⁺T cells, and PD-1-negative, Tim-3-negative CD8⁺T cells (Table 4). Cytokine production ability refers to cytokine production ratios of three kinds of cytokines (A), IFNγ/TNFα/IL-2, two kinds of cytokines (B), IFNγ/TNFα, two kinds of cytokines (C), FNγ/IL-2, and one kind of cytokine (D), IFNγ.

The results shown in Table 4 revealed that cytokine production ability for three kinds of cytokines was significantly different between healthy subjects and cancer patients in the total CD8⁺T cells, PD-1-positive, Tim-3-negative CD8⁺T cells, and PD-1-negative, Tim-3-negative CD8⁺T cells. More specifically, by the metformin treatment, decrease in multifunctionality was very small (zero) in cancer patients. For cytokine production ability for two kinds of cytokines, there was no clear difference between healthy subjects and cancer patients. Further, also for cytokine production ability for one kind of cytokine, there was no clear difference between healthy subjects and cancer patients.

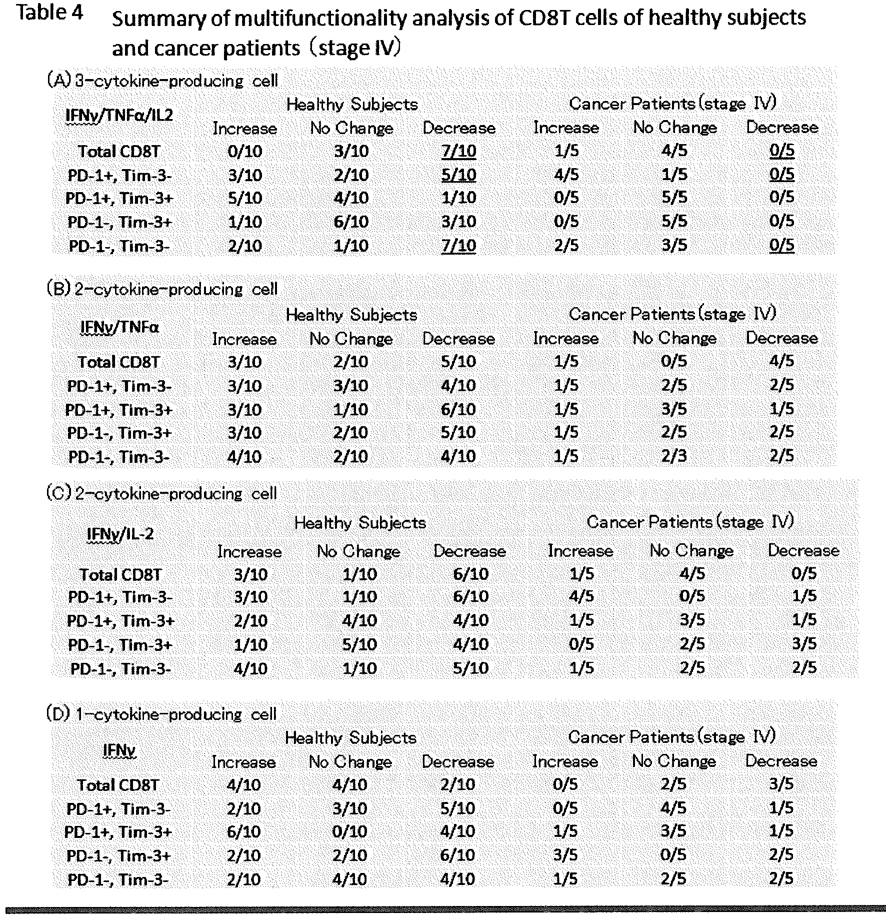

With the results of Examples 3 and 4 showing the test results of multifunctionality recovery by metformin in healthy subjects, cancer patients (stage I), and cancer patients (stage IV), analysis was performed focusing on the groups that showed an increase or no change in the production of three kinds of cytokines (IFNγ/TNFα/IL-2) in the total CD8⁺T cells. The production of three kinds of cytokines (IFNγ/TNFα/IL-2) was increased or not changed in 30% of healthy subjects, 61.3% of cancer patients (stage I), and 100% of cancer patients (stage IV), and a significant difference was observed between healthy subjects and cancer patients (Table 5). In cancer patients, it is assumed that potential increase of exhausted CD8⁺T cells occurs along with progression of the cancer stage, and this exhaustion cell group is detected with a status of "increase" or "no change" in multifunctionality by a metformin treatment.

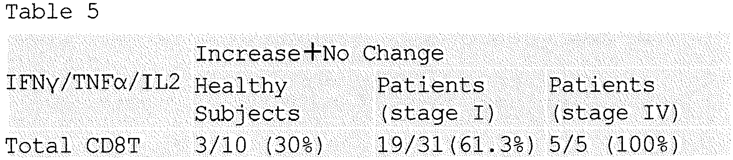

### Example 5: Effects of Metformin Treatment of Cells Ex Vivo

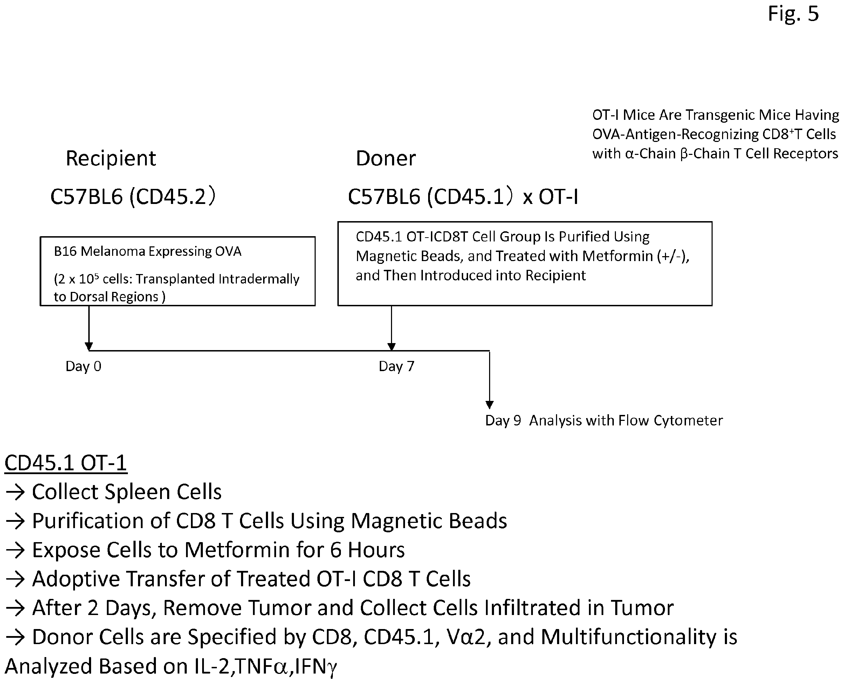

Using mice (C57BL/6), T cells were metformin-treated extracorporeally, and after the cells were placed back into the body, the presence or absence of antitumor effect of the resulting cells was confirmed. First, 8- to 9-week old C57BL/6(CD45.2) mice were used as recipients, and 2×10⁵ of melanoma cell strain (B16-OVA) was transplanted intradermally to the dorsal regions of the recipients. 8- to 9-week old C57BL/6(CD45.1)×OT-1 mice (transgenic mice having OVA-antigen-recognizing CD8⁺T cells with α-chain β-chain T cell receptors) were used as donors. A CD45.1 OT-1 CD8⁺T cell group obtained from a removed spleen was purified using magnetic beads. After the cells were metformin-treated (+/-), the cells were introduced into recipients seven days after the tumor cell transplantation (see Fig. 5). More specifically, CD8⁺T cells were cultured for 6 hours in metformin at a metformin concentration of 3×10⁶/2 mL 10 µM, and at 37°C, 5% CO₂. After the cells (MTi cell) were isolated, the cells were washed twice with PBS, and 3×10⁶ cells were injected from the tail vein of the mouse. A medium obtained by adding Penicillin (50 U/mL), Streptomycin (50 g/mL, Sigma P0781), Sodium Pyruvate (1 mM, Sigma S8636), L-glutamine (2 mM, Sigma G7513), MEM Non-Essential Amino Acids (solution diluted 100 folds, life technologies), 2-mercaptoethanol (5×10⁻⁵ M, Sigma M6250) to RPMI-1640 Medium (Sigma R0883) was used. Fetal bovine serum was not used.

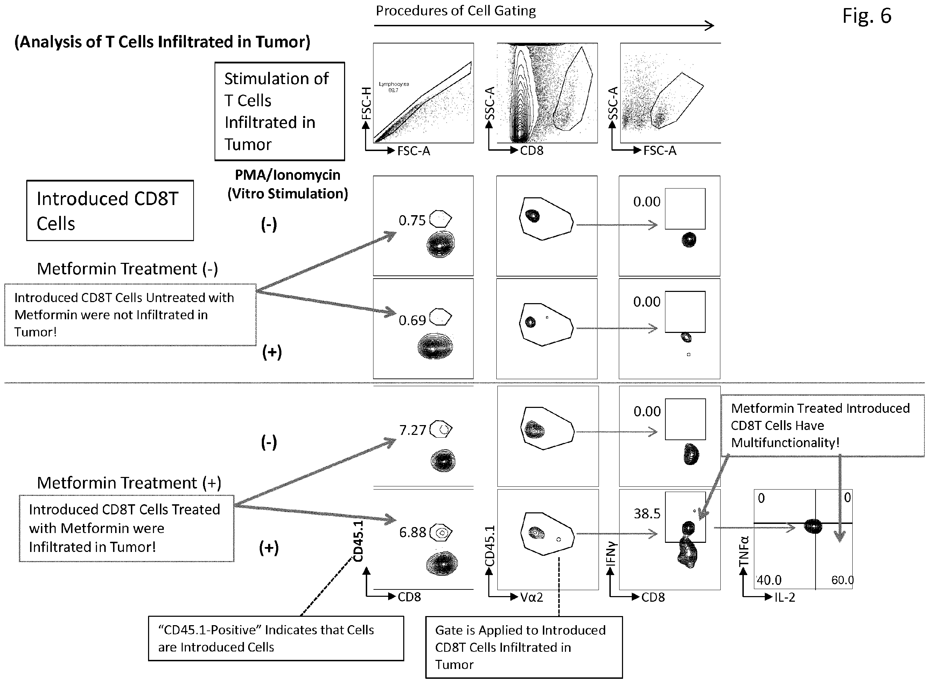

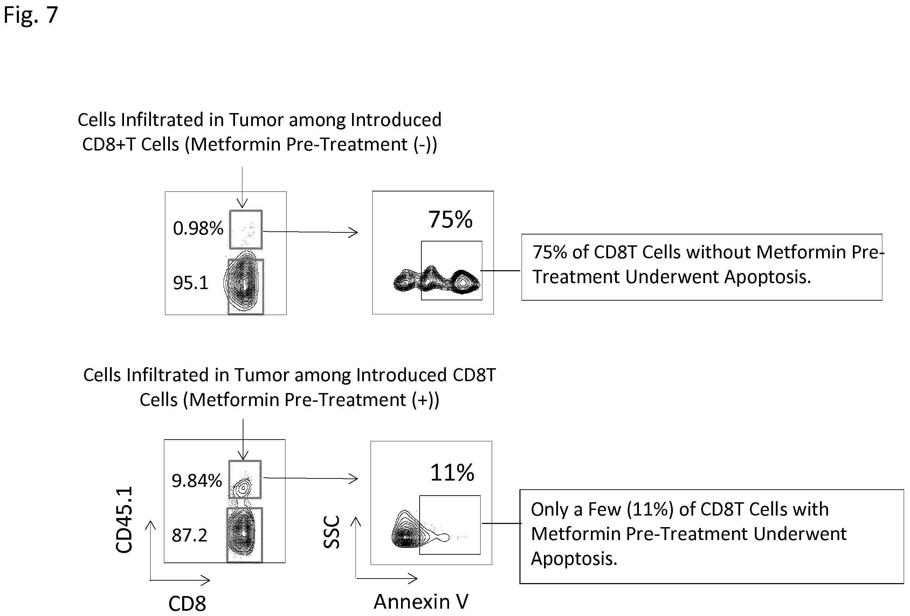

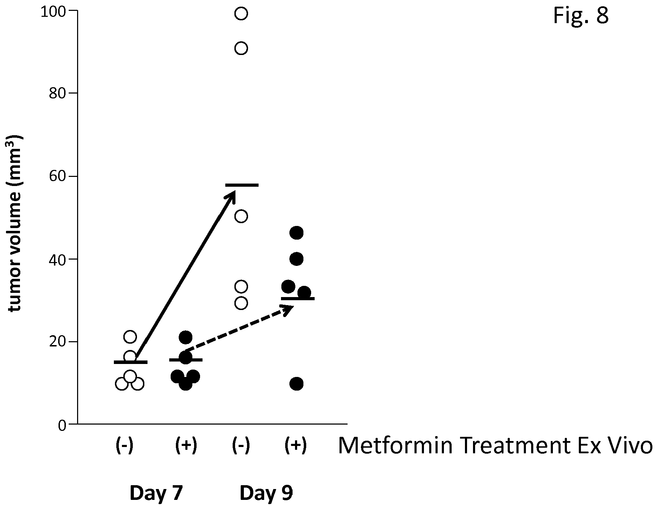

A gate was applied to the resulting CD45.1 OT-1 CD8⁺T cells infiltrated in the tumor by using a FACSCanto™ II flow cytometer (Becton, Dickinson and Company), thereby confirming IFNα and IL-2 production abilities. High cell-positivity ratios for all cytokines were confirmed in the CD45.1 OT-1 CD8⁺T cell (MTi cell) group stimulated by PMA and ionomycin and treated with metformin (see Fig. 6). It was confirmed that the frequency of apoptosis of cells infiltrated in tumors of the recipients after metformin-treated CD45.1 OT-1 CD8⁺T cells (MTi cell) were introduced was significantly lower than the frequency of apoptosis of cells infiltrated in tumors of the recipients after metformin-untreated CD45.1 OT-1 CD8⁺T cells (MTi cell) were introduced (see Fig. 7). On the second day after the introduction of the cells, a significant difference in terms of tumor size was observed between the recipients in which metformin-treated CD45.1 OT-1 CD8⁺T cells (MTi cells) were introduced and the recipients in which metformin-untreated CD45.1 OT-1 CD8⁺T cells were introduced (see Fig. 8). It was confirmed that the efficiency of transfer to tumors of the introduced metformin-treated CD45.1 OT-1 CD8⁺T cells was high, and that these CD8⁺T cells also have high multifunctionality in tumors. The results confirmed that the T cells that were extracorporeally metformin-treated and then placed back into the body showed an antitumor effect, thereby confirming that cell immunotherapy is possible.

### Example 6: Effects of Metformin Treatment of Cells Ex Vivo

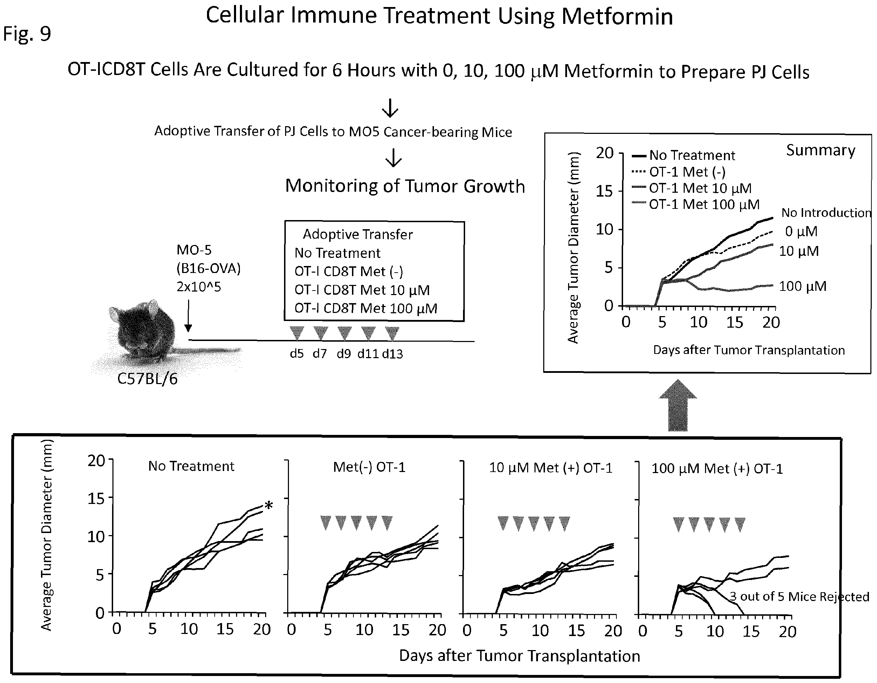

The presence or absence of antitumor effect of T cells that were extracorporeally treated with metformin and then placed back into the body was confirmed using mice (C57BL/6). First, 8-to 9-week old C57BL/6(CD45.2) were used as recipients, and 2×10⁵ of melanoma cell strain (B16-OVA) was transplanted intradermally to the dorsal regions of recipients. CD8⁺T cells of donor OT-I mice (Cell, Vol. 76, pp.17-27, 1994) treated with metformin (0 µM, 10 µM, 100 µM) were introduced into the recipients multiple times on the 5th, 7th, 9th and 11th day after tumor cell transplantation, and tumor growth curve was monitored (see Fig. 9).

The results confirmed that excellent tumor inhibitory effects were observed in the tumors of the recipients in which CD8⁺T cells (MTi cells) of donor OT-1 mice treated with 100 µM metformin were introduced (see Fig. 9).

### Example 7: Effects of Combined Use of Metformin Oral Administration and Cancer Vaccine Administration

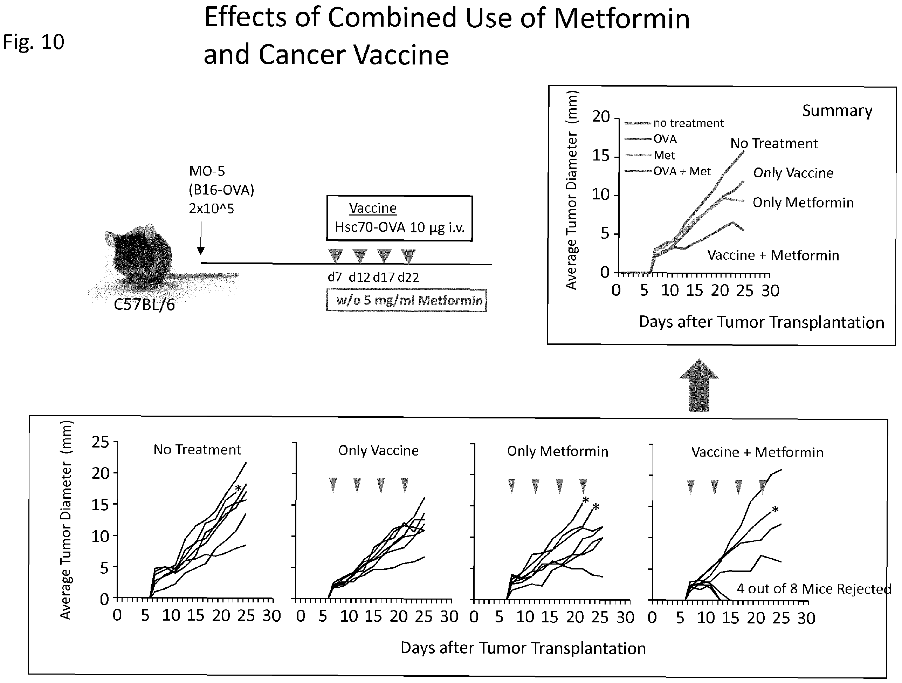

Antitumor effects when free-water-drinking metformin oral administration was performed together with a treatment with OVA vaccine (a fusion protein obtained by fusing OVA₂₅₇₋₂₆₄ with the C terminus of mouse hsc70: Int. Immunol. 13, 1233-1242, 2001) were confirmed. On the 7th, 12th, 17th, and 22nd days after tumor cell transplantation, free-water-drinking metformin administration and/or a treatment with OVA vaccine was performed via the tail veins of the recipients (see Fig. 10). The cancer vaccine of this Example belongs to the category of cancer protein vaccines.

The results confirmed that excellent tumor inhibitory effects were observed in the tumors of the recipients received a combination of metformin and cancer vaccine (see Fig. 10).

### Example 8: Effects of Combined Use of Metformin Administration and Anti-PD-1 Antibody Administration

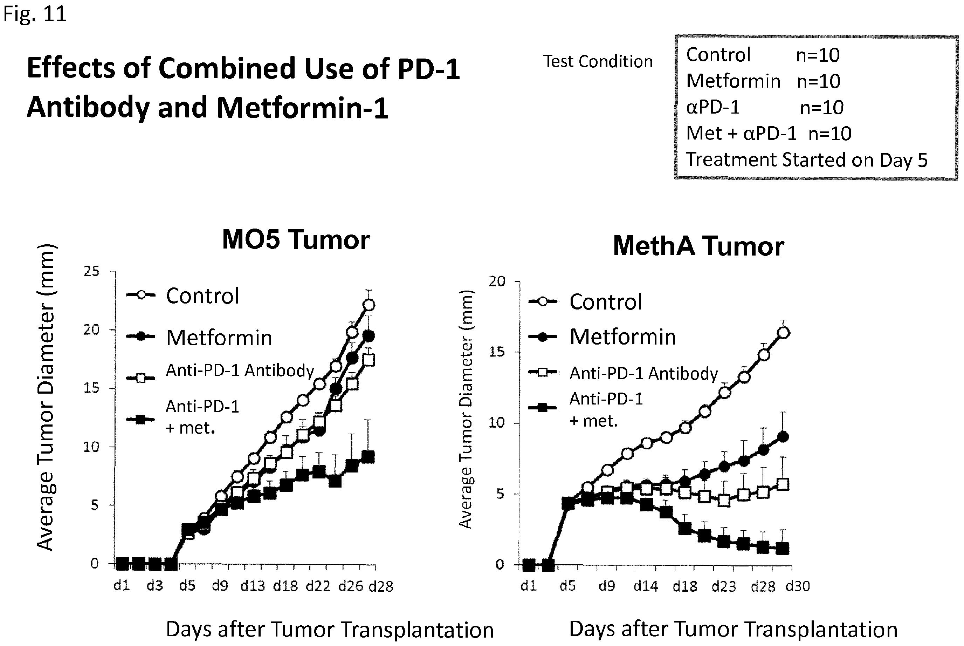

Tumors were transplanted to recipient mice, and effects of combined use of metformin administration and anti-PD-1 antibody administration were confirmed. 8- to 9-week old C57BL/6(N=10) or BALB/c(N=10) were used as mice. MO5 as tumor cells were intradermally transplanted to the dorsal regions of C57BL/6, and Meth A to the dorsal regions of BALB/c, both in amounts of 2×10⁵. A tumor growth curve upon free-water-drinking metformin administration and/or anti-PD-1 antibody (clone 4H2, a murinized chimeric antibody with a variable region of rat-derived anti-mouse PD-1 antibody and a constant region of mouse IgGκ1 (antibody produced by the method of Example 12 in WO2006/121168): αPD-1, provided by Ono Pharmaceutical Co., Ltd.) administration five days after tumor cell transplantation was monitored. The anti-PD-1 antibody was intraperitoneally administered in an amount of 200 µg on the 5th, 11th, 17th, and 23rd days after the tumor cell transplantation. PBS was administered to the control. Fig. 11 shows average tumor diameter of 10 mice as one group. The results confirmed clear tumor shrinkage in the group with combined administration of metformin and anti-PD-1 antibody.

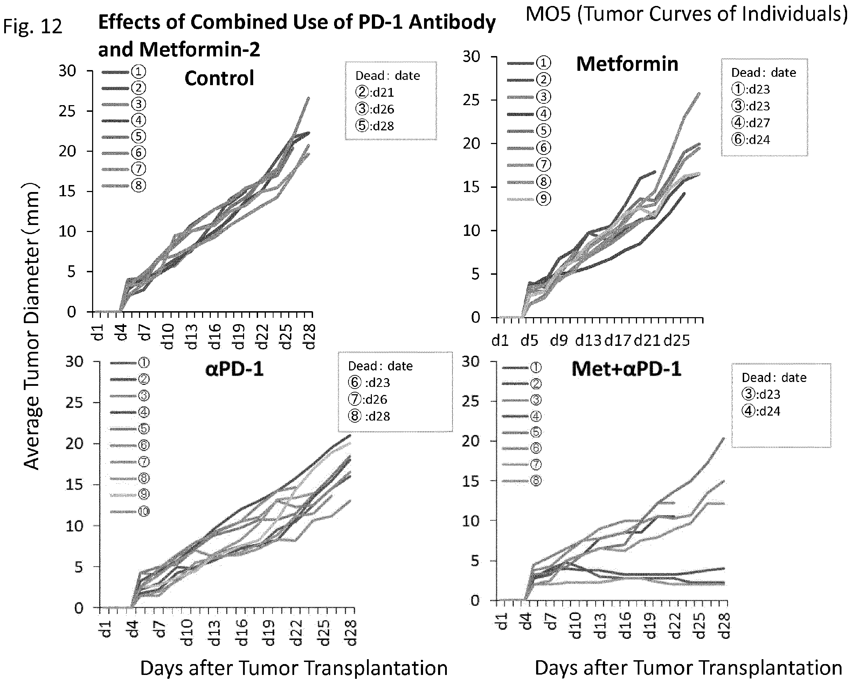

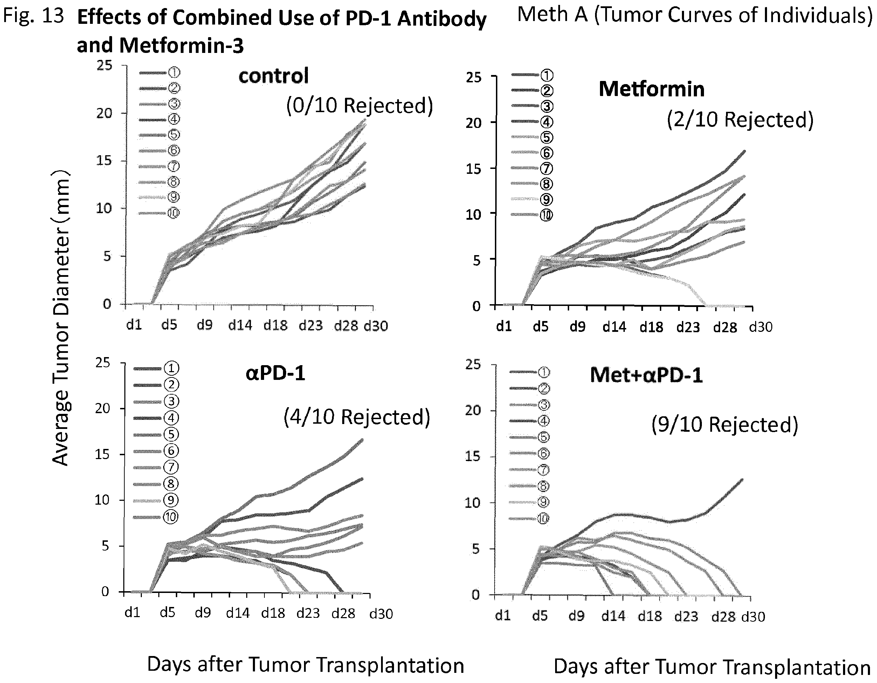

Fig. 12 shows measurement of tumor diameters of C57BL/6 in the control group, the group with administration of metformin alone, the group with administration of αPD-1 alone, and the group with combined administration of metformin and αPD-1. Similarly, Fig. 13 shows measurement of tumor diameters of BALB/c. These results confirmed apparent tumor shrinkage in the group with combined administration of metformin and anti-PD-1 antibody.

### Industrial Applicability

As described above in detail, measurement of the cell-positivity ratio with regard to three kinds of cytokines enables easy examination of immune status of the patient. Further, the evaluation method of the present invention enables combining detecting exhaustion molecules in immune cells and measuring the cell-positivity ratio with regard to three kinds of cytokines, thereby enabling easy and accurate examination of immune status of the patient.

In previous cancer vaccine treatments, the patient to be treated was selected based on the presence or absence of expression of vaccine antigen in the cancer tissues; however, since the evaluation method of the present invention provides information on immune status before immunotherapy, it makes it easier to select patients subjected to vaccine treatment, in addition to antigen expression. This is great contribution to the patients to be treated.

The agent for treating diseases associated with immune abnormalities of the present invention improves immunity, and, for example, for cancers, the agent is expected to improve the prognosis by being combined with surgery, radiation treatment, or chemotherapy. Further, the effects in use as preoperative chemotherapy are considered to be significant. Further, effects of suppressing recurrence after treatment can be expected.

Further, the present invention also encompasses a method for enhancing immune cell function, and further encompasses immune cells with enhanced function (MTi cells) and an agent for treating and/or preventing diseases associated with immune abnormalities containing the MTi cells as an active ingredient. The obtained cells have a superior immune function and may be used as an agent for treating diseases associated with immune abnormalities. For example, the cells may exhibit a superior effect as an antitumor agent.

## Claims

1. A method for enhancing immune cell function, comprising treating removed immune cells in vitro using a therapeutic agent comprising, as an active ingredient, a biguanide antidiabetic drug selected from metformin, phenformin, and buformin.

2. The method for enhancing immune cell function according to claim 1, wherein the immune cells are selected from CD8⁺T cells, CD4⁺T cells, NK cells, γδT cells, NKT cells, B cells, and bone marrow cells.

3. Immune cells that are enhanced in function by the method of claim 1 or 2.

4. An agent for treating and/or preventing diseases associated with immune abnormalities, comprising, as an active ingredient, immune cells that are enhanced in function by the method of any one of claims 1 to 3.

5. The agent for treating and/or preventing diseases associated with immune abnormalities according to claim 4, wherein the diseases associated with immune abnormalities are selected from cancers, infections, and autoimmune diseases.

6. A method for evaluating multifunctionality of removed immune cells, comprising treating removed immune cells in vitro using a therapeutic agent comprising, as an active ingredient, a biguanide antidiabetic drug selected from metformin, phenformin, and buformin; measuring cytokine production ability for three kinds of cytokines, IL-2, TNFα, and IFNγ, of the treated immune cells; and measuring a cell-positivity ratio representing a ratio of cells in which cytokine production abilities with respect to the three kinds of cytokines are all positive.

7. The multifunctionality evaluation method according to claim 6, further comprising the step of measuring expressions of Tim-3 and/or PD-1 in the removed immune cells.

8. The multifunctionality evaluation method according to claim 6 or 7, wherein the immune cells are selected from CD8⁺T cells, CD4⁺T cells, NK cells, γδT cells, NKT cells, B cells, and bone marrow cells.

9. A test method for diseases associated with immune abnormalities, the method using the multifunctionality evaluation method of any one of claims 6 to 8.

10. The test method according to claim 9, wherein the test for diseases associated with immune abnormalities is a test supplementarily used to determine prediction of efficacy of a therapeutic agent for the disease, prediction of severity of the disease, prediction of prognosis of the disease, or prediction of onset of the disease.

11. The test method according to claim 10, wherein the therapeutic agent for the disease is a therapeutic agent comprising, as an active ingredient, a biguanide antidiabetic drug selected from metformin, phenformin, and buformin.

12. An agent for treating and/or preventing diseases associated with immune abnormalities, comprising, as an active ingredient, a biguanide antidiabetic drug selected from metformin, phenformin, and buformin, the agent being used after a test using the test method according to any one of claims 9 to 11.

13. The agent for treating and/or preventing diseases associated with immune abnormalities according to claim 12, wherein the diseases associated with immune abnormalities are selected from cancers, infections, and autoimmune diseases.

14. An agent for treating and/or preventing diseases associated with immune abnormalities comprising, as an active ingredient, a biguanide antidiabetic drug selected from metformin, phenformin, and buformin, the agent being administered in combination with at least one therapeutic agent selected from immunosuppressive factor blocking agent and costimulatory receptor agonist.

15. An agent for treating and/or preventing diseases associated with immune abnormalities, comprising, as an active ingredient, at least one therapeutic agent selected from immunosuppressive factor blocking agent and costimulatory receptor agonist, the agent being administered in combination with a biguanide antidiabetic drug selected from metformin, phenformin, and buformin.

16. The agent for treating and/or preventing diseases associated with immune abnormalities according to claim 14 or 15, wherein the immunosuppressive factor blocking agent is one or any combination of antibodies or fusion proteins selected from anti-CTLA-4 antibody, anti-PD-1 antibody, anti-PD-L1 antibody, anti-PD-L2 antibody, PD-L1 fusion protein, PD-L2 fusion protein, anti-Tim-3 antibody, anti-LAG-3 antibody, anti-BTLA antibody, anti-VISTA antibody.

17. The agent for treating and/or preventing diseases associated with immune abnormalities according to claim 16, wherein the anti-CTLA-4 antibody is Ipilimumabor or Tremelimumab, the anti-PD-1 antibody is Nivolumab, Pembrolizumab, PDR-001, REGN-2810, BGB-A317, or AMP-514, and the anti-PD-L1 antibody is Atezolizumab, Avelumab, Durvalumab, or BMS-936559, and the PD-L2 fusion protein is AMP-224.

18. The agent for treating and/or preventing diseases associated with immune abnormalities according to claim 14 or 15, wherein the costimulatory receptor agonist is an antibody or any combination of antibodies selected from anti-CD137 antibody, anti-OX40 antibody, anti-HVEM antibody, anti-CD27 antibody, anti-GITR antibody, and anti-CD28 antibody.

19. An agent for treating and/or preventing diseases associated with immune abnormalities, comprising as an active ingredient, a biguanide antidiabetic drug selected from metformin, phenformin, and buformin, and is administered in combination with a cancer vaccine.

20. The agent for treating and/or preventing diseases associated with immune abnormalities according to claim 19, wherein the cancer vaccine is a cancer peptide vaccine, a cancer protein vaccine, a dendritic cell vaccine, or a vaccine against a cancer proved to be developed by viral infection.

21. The agent for treating and/or preventing diseases associated with immune abnormalities according to any one of claims 13 to 20, wherein the diseases associated with immune abnormalities are selected from cancers, infections, and autoimmune diseases.

22. The agent for treating and/or preventing diseases associated with immune abnormalities according to claim 21, wherein the cancer is at least one cancer selected from head and neck cancers, esophagus cancer, gastric cancer, colorectal cancer, colon cancer, rectum cancer, liver cancer, gallbladder cancer, cholangiocarcinoma, biliary tract cancer, pancreatic cancer, lung cancer, breast cancer, ovarian cancer, cervical cancer, endometrial cancer, vaginal cancer, vulvar cancer, renal cancer, urothelial cancer, prostate cancer, testicular tumor, osteosarcoma, soft-tissue sarcoma, leukemia, myelodysplastic syndrome, malignant lymphoma, adult T-cell leukemia, multiple myeloma, skin cancer, brain tumor, pleural mesothelioma, and unknown primary cancer.

23. The agent for treating and/or preventing diseases associated with immune abnormalities according to claim 21 or 22, wherein the cancer treatment is suppression of cancer progression, and the cancer prevention is prevention of cancer recurrence.

24. An agent for suppressing progression, treating, preventing, and/or preventing recurrence of cancer, comprising, as an active ingredient, anti-PD-1 antibody, the agent being administered in combination with metformin, wherein the cancer is at least one cancer selected from head and neck cancers, esophagus cancer, gastric cancer, colorectal cancer, hepatocellular cancer, pancreatic cancer, non-small cell lung cancer, small cell lung cancer, breast cancer, ovarian cancer, cervical cancer, endometrial cancer, vaginal cancer, vulvar cancer, renal cell cancer, urothelial cancer, Hodgkin's lymphoma, follicular lymphoma, diffuse large B-cell lymphoma, multiple myeloma, malignant melanoma, glioblastoma and pleural mesothelioma.

25. The agent for treating and/or preventing diseases associated with immune abnormalities according to any one of claims 14 to 24, wherein the agent is used after a test using the test method of any one of claims 9 to 11.
